(19) Europäisches Patentamt
European Patent Office
Office européen des brevets

(11) Veröffentlichungsnummer: **0 464 465 B1**

(12) # EUROPÄISCHE PATENTSCHRIFT

(45) Veröffentlichungstag der Patentschrift: **11.01.95**

(21) Anmeldenummer: **91110016.2**

(22) Anmeldetag: **19.06.91**

(51) Int. Cl.[6]: **C07C 225/22**, A61K 31/135, C07C 255/58, A61K 31/275, C07D 237/30, C07C 215/30, C07C 217/48

(54) **Substituierte Aminoalkylbiphenylderivate, antimykotische Präparate damit und Zwischenprodukte für ihre Herstellung.**

(30) Priorität: **29.06.90 CH 2177/90**
**27.03.91 CH 942/91**

(43) Veröffentlichungstag der Anmeldung:
**08.01.92 Patentblatt 92/02**

(45) Bekanntmachung des Hinweises auf die Patenterteilung:
**11.01.95 Patentblatt 95/02**

(84) Benannte Vertragsstaaten:
**AT BE CH DE DK FR GB IT LI LU NL SE**

(56) Entgegenhaltungen:
**GB-A- 2 185 980**

(73) Patentinhaber: **F. HOFFMANN-LA ROCHE AG**
**Postfach 3255**
**CH-4002 Basel (CH)**

(72) Erfinder: **Guerry, Philippe, Dr.**
**Burgfelderstrasse 30**
**CH-4055 Basel (CH)**
Erfinder: **Jolidon, Synèse, Dr.**
**Schillerstrasse 5**
**CH-4127 Birsfelden (CH)**

(74) Vertreter: **Grossner, Lutz, Dr. et al**
**Grenzacher Strasse 124**
**Postfach 3255**
**CH-4002 Basel (CH)**

EP 0 464 465 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Vorliegende Erfindung betrifft Verbindungen der allgemeinen Formel

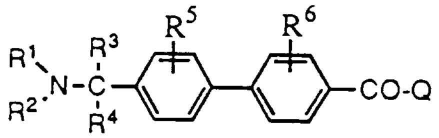

I

worin $R^1$ und $R^2$ je Wasserstoff, $C_{1-7}$-Alkyl oder $c_{2-7}$-Alkenyl oder zusammen geradkettiges $C_{2-4}$-Alkylen, $R^3$ und $R^4$ Wasserstoff oder $C_{1-7}$-Alkyl, $R^5$ und $R^6$ Wasserstoff, Halogen, Trifluormethyl, Nitro, Cyano, $C_{1-7}$-Alkoxy oder $C_{1-7}$-Alkyl; und Q eine unsubstituierte oder durch Halogen, Trifluormethyl, Cyano, Nitro, $C_{1-7}$-Alkyl oder $C_{1-7}$-Alkoxy einfach oder mehrfach substituierte Phenyl- oder Naphthylgruppe, oder eine $C_{1-10}$-Alkylgruppe, die durch eine oder gegebenenfalls mehrere Hydroxygruppen substituiert sein kann, oder eine $C_{1-10}$-Alkenylgruppe bedeuten,
und ihre pharmazeutisch annehmbaren Säureadditionssalze.

Diese Verbindungen sind neu und besitzen wertvolle pharmakologische Eigenschaften. Sie besitzen insbesondere eine ausgeprägte antimykotische Wirkung und zeigen synergistische Effekte in Kombination mit bekannten, antimykotisch wirksamen Substanzen, welche die Sterol-Biosynthese hemmen, wie Ketoconazol und Terbinafin. Die Verbindungen der Formel I können demnach als Heilmittel verwendet werden, insbesondere zur Bekämpfung oder Verhütung von topischen oder systemischen Infektionen, welche durch pathogene Pilze, verursacht werden.

Gegenstand der vorliegenden Erfindung sind: Die obigen Verbindungen der Formel I und ihre pharmazeutisch annehmbaren Säureadditionssalze als solche und zur Anwendung als therapeutische Wirkstoffe; Arzneimittel auf der Basis dieser Stoffe und deren Herstellung; die Verwendung dieser Stoffe als Heilmittel und zur Herstellung von antifungal wirksamen Arzneimitteln; sowie die Herstellung der Verbindungen der Formel I und ihre pharmazeutisch annehmbaren Säureadditionssalze und Zwischenprodukte zu deren Herstellung.

Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Alkenyl" bezeichnet geradkettige oder verzweigte Kohlenwasserstoffreste mit einer olefinischen Doppelbindung, wie Allyl und 2-Butenyl. Der Ausdruck "Alkoxy" bezeichnet über ein Sauerstoffatom gebundene Alkylgruppen, wie Methoxy und Aethoxy. Der Ausdruck "Alkylen" bezeichnet geradkettige oder verzweigte, gesättigte Kohlenwasserstoffreste mit zwei freien Valenzen, wie Dimethylen, Trimethylen und Tetramethylen. Der Ausdruck "Halogen" bezeichnet die vier Formen Fluor, Chlor, Brom und Jod.

Der weiter unten verwendete Ausdruck "Abgangsgruppe" bezeichnet Halogenatome, insbesondere Chlor. Brom und Jod, Trifluormethylsulfonyloxy, und niedere Alkyl- und Arylsulfonyloxygruppen, wie Methylsulfonyloxy, Benzolsulfonyloxy, p-Toluolsulfonyloxy und p-Chlorphenylsulfonyloxy.

$R^1$ und $R^2$ bedeuten vorzugsweise $C_{1-4}$-Alkyl oder $C_{3-4}$-Alkenyl. $R^3$ bedeutet vorzugsweise Wasserstoff oder $C_{1-4}$-Alkyl. $R^4$ bedeutet vorzugsweise Wasserstoff. $R^5$ und $R^6$ bedeuten vorzugsweise Wasserstoff, Halogen oder $C_{1-4}$-Alkyl, insbesondere Wasserstoff. Q bedeutet vorzugsweise eine unsubstituierte oder durch Halogen, insbesondere Brom, Chlor oder Fluor, Trifluormethvl, Nitro, Cyano oder $C_{1-4}$-Alkyl mono- oder disubstituierte Phenylgruppe, oder eine $C_{5-10}$-Alkyl, $C_{5-10}$-Hydroxyalkyl- oder $C_{5-10}$-Alkenylgruppe.

Im Rahmen der vorliegenden Erfindung besonders bevorzugte Verbindungen der Formel I sind:
4'-[1-(Dimethylamino)äthyl]-4-biphenylylphenylketon,
4-Bromphenyl-4'-[(dimethylamino)methyl]-4-biphenylylketon,
4'-[(Dimethylamino)methyl-4-biphenylyl-4-jodphenylketon,
4'-[1-(Allylmethylamino)äthyl]-4-biphenylylphenylketon,
4'-[1-(Allylmethylamino)methyl]-4-biphenylyl-4-bromphenylketon,
4'-[1-(Allylmethylamino)methyl]-4-biphenylyl-4-jodphenylketon,
2,4-Difluorophenyl-4'-[(allyl-methylamino)methyl]-4-biphenylketon,
4'-[(Dimethylamino)methyl]-4-biphenyl-4-methyl-3-pentenylketon,
4'-[(Allyl-methylamino)methyl]-4-biphenylyl-4-methyl-3-pentenylketon und
4'-[(Allyl-methylamino)methyl]-4-biphenylyl-8-hydroxyoctylketon.

Die Verbindungen der Formel I und ihre pharmazeutisch annehmbaren Säureadditionssalze können erfindungsgemäss hergestellt werden, indem man

a) eine Verbindung der allgemeinen Formel

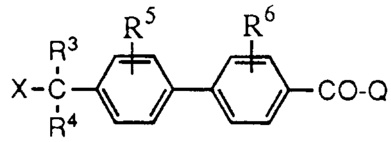

II

worin X eine Abgangsgruppe bedeutet, und $R^3$, $R^4$, $R^5$, $R^6$ und Q obige Bedeutung besitzen, mit einem Amin der allgemeinen Formel $HNR^1R^2$, worin $R^1$ und $R^2$ obige Bedeutung besitzen, umsetzt, oder

b) eine Verbindung der allgemeinen Formel

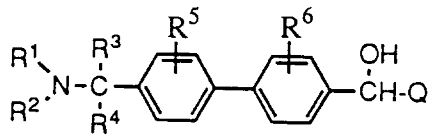

III

worin $R^1$, $R^2$, $R^3$, $R^4$, $R^5$, $R^6$ und Q obige Bedeutung besitzen, oxidiert, oder

c) eine Verbindung der allgemeinen Formel

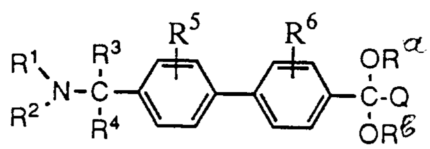

IV

worin $R^a$ und $R^b$ $C_{1-4}$-Alkyl oder zusammen Dimethylen oder Trimethylen bedeuten, und $R^1$, $R^2$, $R^3$, $R^4$, $R^5$, $R^6$ und Q obige Bedeutung besitzen, mit einer wässrigen Säure behandelt, oder

d) eine Verbindung der allgemeinen Formel

X'-CO-Q    V

worin X' Halogen bedeutet, und Q obige Bedeutung besitzt, in Gegenwart einer Lewis-Säure mit einer Verbindung der allgemeinen Formel

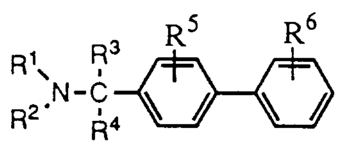

VI

worin $R^1$, $R^2$, $R^3$, $R^4$, $R^5$ und $R^6$ obige Bedeutung besitzen, umsetzt, oder

e) eine Verbindung der allgemeinen Formel

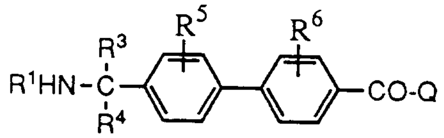

Ia

worin $R^1$, $R^3$, $R^4$, $R^5$, $R^6$ und Q obige Bedeutung besitzen, alkyliert oder alkenyliert,

f) eine Verbindung der allgemeinen Formel

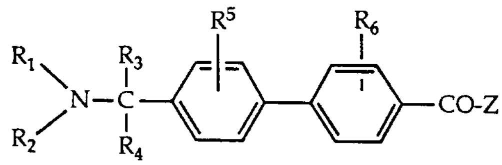

XXIV

worin $R^1$, $R^2$, $R^3$, $R^4$, $R^5$, $R^6$ obige Bedeutung besitzen, und Z eine Abgangsgruppe der allgemeinen Formel $NR^cR^d$ bedeutet, worin $R^c$ niederes Alkyl und $R^d$ niederes Alkyl oder Alkoxygruppe bedeuten, mit einer Verbindung der allgemeinen Formel M-Q worin M -MgCl, -MgBr, -MgI oder -Li bedeutet, und Q die obige Bedeutung besitzt,

umsetzt, und

g) eine erhaltene Verbindung der Formel I erwünschtenfalls in ein pharmazeutisch annehmbares Säureadditionssalz überführt.

Die Umsetzung einer Verbindung der Formel II mit einem Amin der Formel $HNR^1R^2$ gemäss Verfahrensvariante a) kann nach an sich bekannten und jedem Fachmann geläufigen Methoden durchgeführt werden. Die Reaktion wird vorzugsweise in einem polaren Lösungsmittel und in Gegenwart einer Base als Säurebindendes Mittel in einem Temperaturbereich von etwa 0 °C bis etwa 150 °C durchgeführt. Geeignete Lösungsmittel sind beispielsweise niedere Alkohole, wie Methanol und Aethanol, und niedere Dialkylketone, wie Aceton. Geeignete Basen sind beispielsweise überschüssiges Amin der Formel $HNR^1R^2$, tertiäre Amine, wie Triäthylamin, und anorganische Basen, wie Alkalimetallcarbonate, -hydroxide und -alkoholate.

Die Oxidation einer Verbindung der Formel III gemäss Verfahrensvariante b) kann nach an sich bekannten und jedem Fachmann geläufigen Methoden durchgeführt werden. Die Reaktion wird vorzugsweise in einem inerten Lösungsmittel und in Gegenwart eines Oxidationsmittels in einem Temperaturbereich von etwa -80 °C bis etwa Raumtemperatur durchgeführt. Geeignete Lösungsmittel sind beispielsweise chlorierte, niedere Kohlenwasserstoffe, wie Methylenchlorid und Chloroform. Geeignete Oxidationsmittel sind beispielsweise Mangandioxid oder Mischungen von Dimethylsulfoxid mit Oxalylchlorid, Dicyclohexylcarbodiimid oder Acetanhydrid und einem tertiären Amin, wie Triäthylamin.

Für die Behandlung einer Verbindung der Formel IV mit einer wässrigen Säure gemäss Verfahrensvariante c) verwendet man vorzugsweise eine verdünnte, wässrige Mineralsäure, z.B. verdünnte Salzsäure, und arbeitet in einem Temperaturbereich von etwa 0 °C bis etwa Raumtemperatur.

Die Umsetzung einer Verbindung der Formel V mit einer Verbindung der Formel VI gemäss Verfahrensvariante d) kann nach an sich bekannten und jedem Fachmann geläufigen Methoden durchgeführt werden. Die Reaktion wird vorzugsweise in einem inerten Lösungsmittel und in Gegenwart einer Lewis-Säure in einem Temperaturbereich von etwa 0 °C bis etwa 100 °C durchgeführt. Geeignete Lösungsmittel sind beispielsweise halogenierte niedere Kohlenwasserstoffe, wie Methylenchlorid, Chloroform und Aethylenchlorid, Nitrobenzol und Schwefelkohlenstoff. Als Lewis-Säure verwendet man vorzugsweise Aluminiumchlorid. Besonders geeignete Verbindungen der Formel V sind die entsprechenden Carbonsäurechloride.

Die Alkylierung bzw. Alkenylierung einer Verbindung der Formel Ia gemäss Verfahrensvariante e) kann nach an sich bekannten und jedem Fachmann geläufigen Methoden durchgeführt werden. Die Reaktion wird vorzugsweise in Gegenwart einer Base, in einem polaren Lösungsmittel und in einem Temperaturbereich von etwa 0 °C bis etwa 100 °C durchgeführt. Geeignete Lösungsmittel sind beispielsweise niedere Alkohole, wie Methanol und Aethanol, niedere Dialkylketone, wie Aceton, Dimethylformamid und Mischungen davon mit Wasser. Als Basen verwendet man vorzugsweise Alkalimetallcarbonate und -hydroxide, wie Kaliumcarbonat und Natriumhydroxid.

Die Umsetzung einer Verbindung der Formel XXIV gemäss Verfahrensvariante f) kann nach an sich bekannten und jedem Fachmann geläufigen Methoden durchgeführt werden. Die Reaktion wird vorzugsweise in einem inerten Lösungsmittel und in einem Temperaturbereich von etwa -80 °C bis etwa Raumtemperatur durchgeführt. Geeignete Lösungsmittel sind beispielsweise offenkettige und cyclische Aether, wie Diäthyläther, Methyl-t-butyläther und Tetrahydrofuran und Mischungen davon.

Die Herstellung von pharmazeutisch annehmbaren Säureadditionssalzen von Verbindungen der Formel I gemäss Verfahrensvariante g) kann nach an sich bekannten und jedem Fachmann geläufigen Methoden durchgeführt werden. Es kommen dabei Salze mit pharmazeutisch annehmbaren anorganischen und organischen Säuren in Betracht. Bevorzugte Säureadditionssalze sind die Hydrochloride, Hydrobromide, Sulfate, Nitrate, Citrate, Acetate, Succinate, Fumarate, Methansulfonate und die p-Toluolsulfonate.

Die verschiedenen als Ausgangsstoffe verwendeten Verbindungen können beispielsweise gemäss den nachfolgenden Reaktionsschemata I-VI und den nachfolgenden Beschreibungen der verschiedenen Reaktionen hergestellt werden. In diesen Reaktionsschemata haben $R^1$, $R^2$, $R^3$, $R^4$, $R^5$, $R^6$, $R^a$, $R^b$, Q, X und X' die obige Bedeutung, M bedeutet -Li, -MgCl, -MgBr oder -MgJ, $R^{31}$ und $R^{41}$ bedeuten niederes Alkyl, X'' bedeutet eine Trifluormethylsulfonyloxy- oder eine niedere Alkyl- oder Arylsulfonyloxygruppe und X''' bedeutet ein Halogenatom oder eine Trifluormethylsulfonyloxygruppe.

Reaktionsschema I

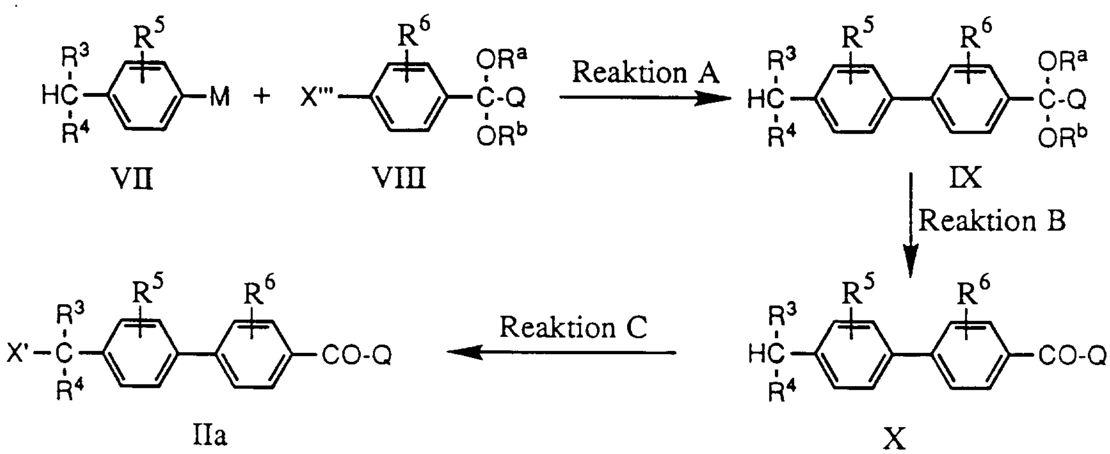


VII VIII IX X IIa

Reaktionsschema II

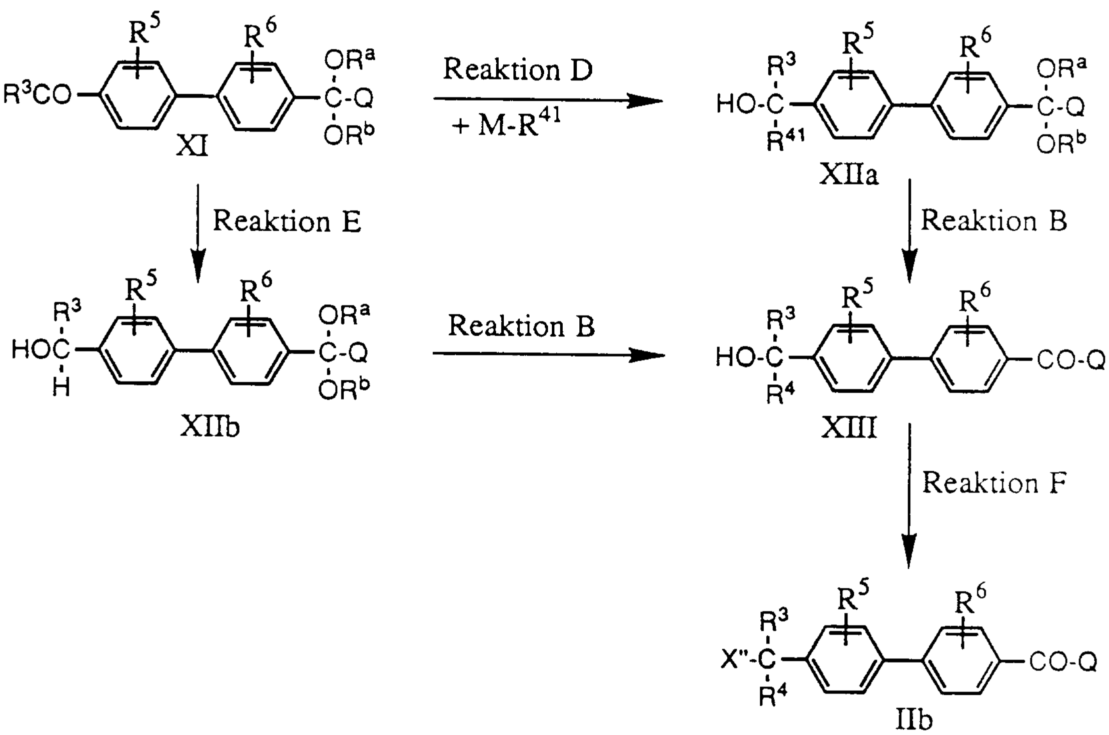


XI XIIa XIIb XIII IIb

Reaktionsschema III

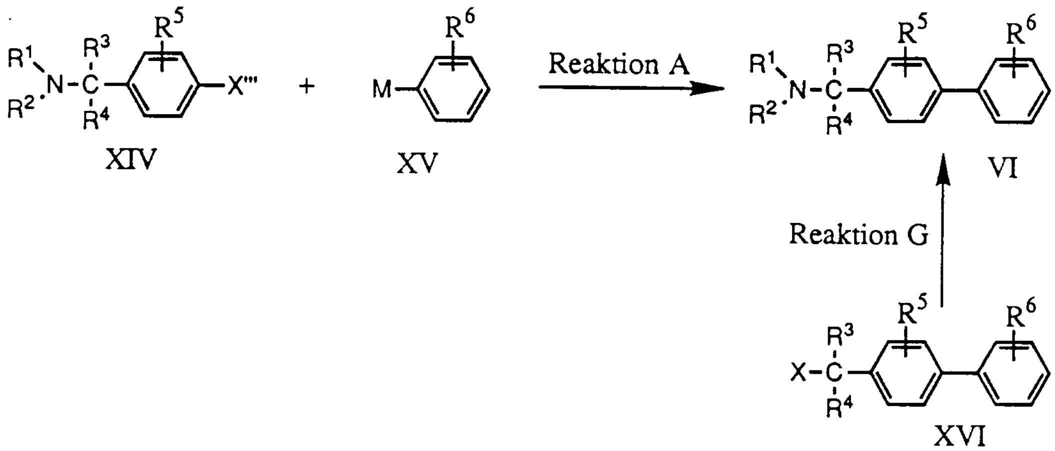


XIV XV VI XVI

Reaktionsschema IV

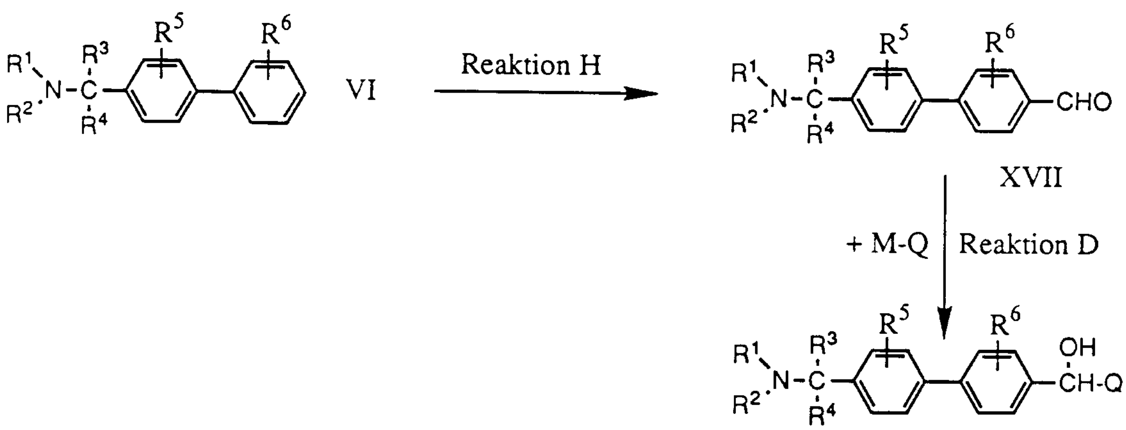


VI XVII III

Reaktionsschema V

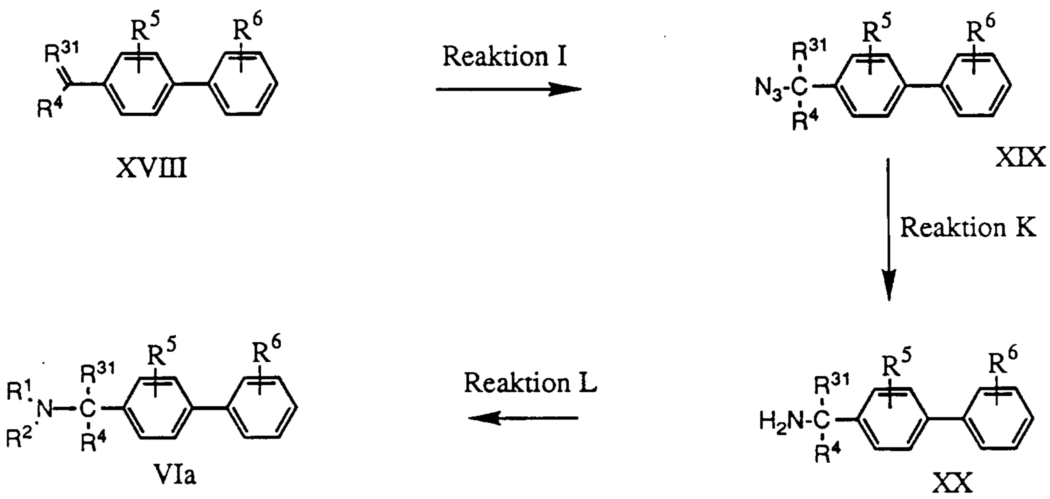


XVIII XIX XX VIa

Reaktionsschema VI

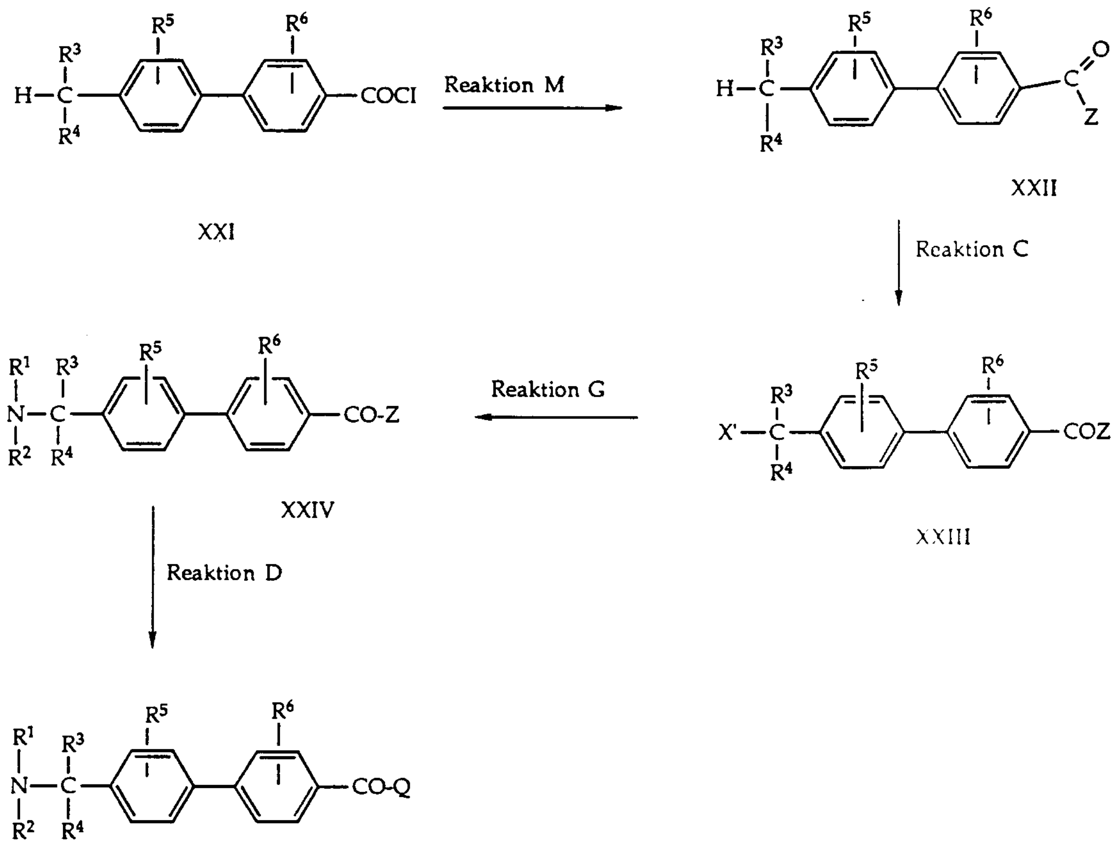


XXI XXII XXIII XXIV

Reaktion A

Diese Reaktion kann nach an sich bekannten und jedem Fachmann geläufigen Methoden durchgeführt werden. Sie wird vorzugsweise in einem inerten Lösungsmittel in einem Temperaturbereich von 20 °C bis

100 °C und in Gegenwart eines geeigneten Katalysators durchgeführt. Das Symbol M bedeutet vorzugsweise -MgBr und X''' bedeutet vorzugsweise Brom. Als Katalysator werden vorzugsweise Pd-Katalysatoren, wie Tetrakis(triphenylphosphin)palladium, oder Ni-Katalysatoren eingesetzt (vgl. D.A. Widdowson und Y.Z Zhang, Tetrahedron 42, 2111 (1986)). Als Lösungsmittel verwendet man vorzugsweise einen offenkettigen oder cyclischen Aether, wie Diäthyläther, Dimethoxyäthan und Tetrahydrofuran.

Reaktion B

Diese Reaktion wird vorzugsweise durch Behandeln mit einer wässrigen Säure durchgeführt. Man verwendet vorzugsweise eine verdünnte, wässrige Mineralsäure, z.B. verdünnte Salzsäure, und arbeitet in einem Temperaturbereich von etwa 0 °C bis etwa Raumtemperatur.

Reaktion C

Diese Halogenierung kann nach an sich bekannten und jedem Fachmann geläufigen Methoden durchgeführt werden. Die Reaktion wird vorzugsweise in einem chlorierten, niederen Kohlenwasserstoff, wie Tetrachlorkohlenstoff oder Chloroform, in einem Temperaturbereich von 0 °C bis 100 °C mit N-Bromsuccinimid oder elementarem Brom oder Chlor durchgeführt. Die Reaktion kann durch Licht oder Radikal-Initiatoren, wie Azaisobutyronitril, katalysiert werden.

Reaktion D

Diese Reaktion kann nach an sich bekannten und jedem Fachmann geläufigen Methoden durchgeführt werden. Sie wird vorzugsweise in einem inerten Lösungsmittel und in einem Temperaturbereich von 0 °C bis 80 °C durchgeführt. Als Lösungsmittel verwendet man vorzugsweise einen offenkettigen oder cyclischen Aether, wie Diäthyläther, Dimethoxyäthan und Tetrahydrofuran.

Reaktion E

Diese Reaktion kann nach an sich bekannten und jedem Fachmann geläufigen Methoden durchgeführt werden. Sie wird vorzugsweise in einem polaren Lösungsmittel, wie Methanol oder Aethanol, und in einem Temperaturbereich von 0 bis 50 °C durchgeführt. Als Reduktionsmittel werden vorzugsweise komplexe Borhydride, wie Natriumborhydrid eingesetzt, oder aber elementarer Wasserstoff in Gegenwart von Uebergangsmetall-Katalysatoren.

Reaktion F

Diese Reaktion kann nach an sich bekannten und jedem Fachmann geläufigen Methoden durchgeführt werden. In einer bevorzugten Ausführungsform verwendet man Pyridin oder Picolin als Lösungsmittel und arbeitet in einem Temperaturbereich von 0 °C bis 50 °C. Als Reagens verwendet man vorzugsweise Trifluormethylsulfonsäureanhydrid oder ein niederes Alkyl- oder Arylsulfonsäurechlorid.

Reaktion G

Diese Reaktion wird durch Umsetzen mit einem Amin der Formel $HNR^1R^2$ durchgeführt. Sie kann nach an sich bekannten und jedem Fachmann geläufigen Methoden durchgeführt werden. Die Reaktion wird vorzugsweise in einem polaren Lösungsmittel und in Gegenwart einer Base als säurebindendes Mittel in einem Temperaturbereich von etwa 0 °C bis etwa 150 °C durchgeführt. Geeignete Lösungsmittel sind beispielsweise niedere Alkohole, wie Methanol und Aethanol, und niedere Dialkylketone, wie Aceton. Geeignete Basen sind beispielsweise überschüssiges Amin der Formel $HNR^1R^2$, tertiäre Amine, wie Triäthylamin, und anorganische Basen, wie Alkalimetallcarbonate, -hydroxide und -alkoholate.

Reaktion H

Diese Formylierung kann nach an sich bekannten und jedem Fachmann geläufigen Methoden durchgeführt werden. Sie wird vorzugsweise in einer starken organischen Säure, wie z.B. Trifluoressigsäure, als Lösungsmittel und in einem Temperaturbereich von 0 °C bis 90 °C durchgeführt. Als Reagenz verwendet man z.B. Hexamethylentetramin, vgl. W.E. Smith, J. org.Chem. 37, 3972 (1972). Die Formylierung kann

aber auch nach Vilsmeyer mit disubstituierten Formamiden wie z.B. N,N-Dimethylformamid, und Phosphoroxychlorid durchgeführt werden, vgl. C. Jutz, Adv. Org. Chem. 9, 225 (1976). Sie kann schliesslich auch mit einem Formylierungsreagenz, wie z.B. Dichlormethylmethyläther oder Orthoameisensäuretriäthylester in Gegenwart einer Lewissäure wie Aluminiumchlorid in einem inerten Lösungsmittel wie Dichlormethan und in einem Temperaturbereich von 0 °C bis 40 °C durchgeführt werden.

Reaktion I

Diese Reaktion wird durch Umsetzen mit einem Alkalimetallazid, vorzugsweise Natriumazid, in an sich bekannter Weise durchgeführt. Als Lösungsmittel verwendet man vorzugsweise einen chlorierten niederen Kohlenwasserstoff, wie Chloroform und 1,2-Dichloräthan. Man arbeitet vorzugsweise in Gegenwart einer starken Säure, wie Trifluoressigsäure und Schwefelsäure, und in einem Temperaturbereich von -10 °C bis 20 °C (vgl. D. Baldermann und A. Kadic, Synthesis 1978, 24).

Reaktion K

Bei dieser Reaktion handelt es sich um eine an sich bekannte Reduktion. In einer bevorzugten Ausführungsform verwendet man Raney-Nickel in einem niederen Alkohol, wie Isopropanol, und arbeitet in einem Temperaurbereich von 0 °C bis 100 °C (vgl. D. Baldermann und A. Kadic, Synthesis 1978, 24).

Reaktion L

Bei dieser Reaktion handelt es sich um eine an sich bekannte Alkylierung bzw. Alkenylierung. Sie kann nach an sich bekannten und jedem Fachmann geläufigen Methoden durchgeführt werden. Die Reaktion wird vorzugsweise in Gegenwart einer Base, in einem polaren Lösungsmittel und in einem Temperaturbereich von etwa 0 °C bis etwa 100 °C durchgeführt. Geeignete Lösungsmittel sind beispielsweise niedere Alkohole, wie Methanol und Aethanol, niedere Dialkylketone, wie Aceton, Dimethylformamid und Mischungen davon mit Wasser. Als Basen verwendet man vorzugsweise Alkalimetallcarbonate und -hydroxide, wie Kaliumcarbonat und Natriumhydroxid.

Reaktion M

Bei dieser Reaktion handelt es sich um eine an sich bekannte Carbonsäureamidierung. Sie kann nach an sich bekannten Methoden durch Umsetzung mit einem Amin der Formel $HNR^cR^d$ oder von einem Salz dieses Amins durchgeführt werden. Als Lösungsmittel verwendet man vorzugsweise einen chlorierten niederen Kohlenwasserstoff, wie Chloroform oder Methylenchlorid. Man arbeitet vorzugsweise in Gegenwart einer organischen Base wie Pyridin oder Triäthylamin, in einem Temperaturbereich von -10 °C bis 20 °C (vgl. S. Nahm und S.M. Weinreb, Tetrahedron Lett. 1981, 22, 3815). Nieder Alkyl- oder Alkoxygruppen $R^c$ und $R^d$ sind vorzugsweise Methyl und Methoxy.

Wie bereits erwähnt besitzen die Verbindungen der Formel I und ihre pharmazeutisch annehmbaren Säureadditionssalze wertvolle antifungale Eigenschaften. Sie sind wirksam gegen eine Vielzahl von pathogenen Pilzen, die topische und systemische Infektionen hervorrufen, wie Candida albicans und Histoplasma capsulatum. Die 2,3-Epoxysqualen-Lanosterol-Cyclase, ein Enzym involviert in der Sterolbiosynthese der eucaryotischen Zelle, ist ein essentielles Enzym für die Pilze. So ist z.B. ein S.cerevisiae Stamm, in welchem dieses Enzym fehlt nicht lebensfähig [F. Karst & F. Lacroute, Molec. Gen. Genet. 154, 269 (1977)-]. Die hemmende Wirkung der Verbindungen der Formel I auf obengenanntes Enzym aus C.albicans wurde als Mass für die antifungale Wirkung genommen. Die Hemmung kann, beispielsweise, mittels der nachfolgend beschriebenen Methode gemessen werden.

Bestimmung des IC50-Wertes für die Hemmung der 2,3-Epoxysqualen-Lanosterol-Cyclase von Candida albicans

Die Zellen einer Kultur von Candida albicans am Ende der logarithmischen Wachstumsphase werden gesammelt und mit 100mM Phosphatpuffer (pH = 6.9), Digestionspuffer und 50mM Phosphatpuffer (pH = 7,4), welcher 1M Mannitol und 5mM DTT enthält, gewaschen.

1,0 g dieser Zellen werden in 5 ml Digestionspuffer aufgeschlämmt, mit 1 mg Zymolase 100T (Seikagaku Kogyo, Japan) und 12,5 µl β-Mercaptoäthanol versetzt und während 30 Minuten bei 30 °C inkubiert. Die entstehenden Protoplasten werden durch Zentrifugation (10 Minuten bei 2500 g) isoliert und

anschliessend durch Zugabe von 2 ml 100mM Phosphatpuffer (pH = 6,9) zum Platzen gebracht. Durch erneute Zentrifugation (10 Minuten bei 10000 g) erhält man einen zellfreien Extrakt (CFE) als Ueberstand. Dieser wird auf 10 mg Protein pro ml verdünnt, und der pH wird auf 6,9 gebracht.

Die Aktivität der 2,3-Epoxysqualen-Lanosterol-Cyclase im CFE wird durch die Umsetzung von $^{14}C$-Squalen-Epoxid in Gegenwart von n-Decylpentaoxyäthylen als Detergens gemessen. Titration mit angemessenen Mengen der Testsubstanz erlauben die Bestimmung des $IC_{50}$-Wertes (Konzentration der Testsubstanz, welche die Enzymaktivität um die Hälfte verringert).

Der Versuch wird wie folgt durchgeführt:

Durch Ultraschallbehandlung bereitet man eine 250µM Lösung von $^{14}C$-Squalen-Epoxid in 100mM Phosphatpuffer (pH = 6,9) unter Zusatz von 1% n-Decylpentaoxyäthylen vor. 100 µl dieser Lösung wird mit 20 µl einer Lösuang der Testsubstanz in Dimethylsulfoxid (bzw. 20 µl reinem Dimethylsulfoxid als Kontrolle) versetzt. Nach Zugabe von 880 µl CFE wird die gut gemischte Lösuang unter Schütteln während 1 Stunde bei 30° inkubiert. Anschliessend wird durch Zugabe von 500 µl 15-proz. Kaliumhydroxid in 90-proz. Aethanol die Reaktion gestoppt.

Das Gemisch wird zweimal mit 1 ml n-Hexan extrahiert, das Hexan wird abgedampft und der Lipid-Rückstand wird in 200 µl Diäthyläther aufgenommen. Nach Dünnschichtchromatographie an Kieselgel mit Methylenchlorid als Laufmittel werden die Platten mit einem Radioaktivitäts-Dünnschicht-Scanner untersucht.

Unter den verwendeten Bedingungen wird ausschliesslich Lanosterol als radioaktives Produkt gefunden. Dessen Menge wird mit der Menge radioaktivem Lanosterol in der Kontrolle verglichen.

Die $IC_{50}$-Werte werden graphisch ermittelt und in µg Testsubstanz pro ml angegeben. Die nachfolgende Tabelle I enthält für repräsentative Vertreter der durch die Formel I definierten Verbindungsklasse die in obigem Versuch ermittelten $IC_{50}$-Werte, sowie Angaben über die akute Toxizität bei subkutaner Verabreichung an Mäusen ($DL_{50}$ in mg/kg).

Tabelle I

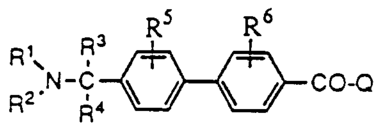

I

| Nr. | $R^1$ | $R^2$ | $R^3$ | $R^4$ | $R^5$ | $R^6$ | Q | $IC_{50}$ in ug/ml | $DL_{50}$ in mg/kg |
|---|---|---|---|---|---|---|---|---|---|
| 1 | $-CH_3$ | $-CH_3$ | H | H | H | H | Ph | 0,100 | |
| 2 | " | " | $-CH_3$ | " | " | " | " | 0,080 | |
| 3 | " | " | H | " | " | " | 4-Fluor-Ph | 0,100 | |
| 4 | " | " | " | " | " | " | 2,4-Dichlor-Ph | 0,077 | |
| 5 | " | " | " | " | " | " | 4-Nitro-Ph | 0,115 | |
| 6 | " | " | " | " | " | " | 4-Brom-Ph | 0,044 | |
| 7 | " | " | " | " | " | 2-Cl | 2,4-Dichlor-Ph | 0,100 | |
| 8 | " | " | " | " | " | H | 2-Chlor-4-Nitro-Ph | 0,044 | |
| 9 | " | " | $-CH_3$ | $-CH_3$ | " | " | Ph | 0,950 | |
| 10 | " | " | H | H | " | " | 4-Jod-Ph | 0,047 | |
| 11 | " | " | " | " | " | " | 4-Cyano-Ph | 0,058 | |
| 12 | " | " | " | " | " | " | 1-Na | 0,310 | |
| 13 | " | " | " | " | " | " | 2-Na | 0,210 | |
| 14 | Allyl | " | " | " | " | " | Ph | 0,018 | >4000 |
| 15 | " | " | " | " | " | " | 4-Fluor-Ph | 0,036 | |
| 16 | " | " | " | " | " | " | 2,4-Dichlor-Ph | 0,016 | |
| 17 | " | " | " | " | " | " | 4-Nitro-Ph | 0,016 | |
| 18 | " | " | $-CH_3$ | " | " | " | Ph | 0,032 | |
| 19 | " | " | H | " | " | " | 4-Cyano-Ph | 0,040 | |
| 20 | " | " | " | " | " | " | 4-Brom-Ph | 0,013 | |
| 21 | " | " | " | " | " | 2-Cl | 2,4-Dichlor-Ph | 0,026 | |
| 22 | " | " | " | " | " | H | 1-Na | 0,170 | |
| 23 | " | " | " | " | " | " | 2-Na | 0,160 | |
| 24 | " | $-CH_3$ | " | " | " | " | 2-Chlor-4-Nitro-Ph | 0,011 | |
| 25 | $-CH_2CH_3$ | | " | " | " | " | Ph | 0,045 | |
| 26 | H | " | " | " | " | " | " | 0,200 | |

| Nr. | $R^1$ | $R^2$ | $R^3$ | $R^4$ | $R^5$ | $R^6$ | Q | $IC_{50}$ in ug/ml | $DL_{50}$ in mg/kg |
|---|---|---|---|---|---|---|---|---|---|
| 27 | $-CH_3$ | " | " | " | " | " | 2,4-Dibrom-Ph | 0,024 | |
| 28 | Allyl | " | " | " | " | " | 2,4-Dibrom-Ph | 0,02 | |
| 29 | $-CH_3$ | " | " | " | " | " | 2-Methoxy-Ph | 0,19 | |
| 30 | " | " | " | " | " | " | 2,4-Difluoro-Ph | 0,029 | |
| 31 | Allyl | " | " | " | " | " | 2,4-Difluoro-Ph | 0,008 | |
| 32 | $-CH_3$ | " | " | " | " | " | 2-Methyl-Ph | 0,03 | |
| 33 | Allyl | " | " | " | " | " | 2-Methyl-Ph | 0,012 | |
| 34 | " | " | " | " | " | " | 2-Methyl-Ph | 0,018 | |
| 35 | $-CH_3$ | " | " | " | " | " | n-Pentyl | 0,031 | |
| 36 | " | " | " | " | " | " | 4-Methyl-3-pentenyl | 0,003 | |
| 37 | " | " | " | " | " | " | 8-Hydroxyoctyl | 0,017 | |
| 38 | Allyl | " | " | " | " | " | n-Pentyl | 0,021 | |
| 39 | Allyl | " | " | " | " | " | 4-Methyl-3-pentenyl | 0,009 | |
| 40 | " | " | " | " | " | " | 8-Hydroxyoctyl | 0,007 | |

Ph = Phenyl

Na = Naphthyl

Die bereits erwähnte synergistische Wirkung der Verbindungen der Formel I und ihrer pharmazeutisch annehmbaren Säureadditionssalze in Kombination mit Sterol-Biosynthese-Hemmern, wie Ketoconazol, kann beispielsweise mittels der Agar-Verdünnungs-Methode gezeigt werden. Man verwendet hierfür Casitonagar und Inokula (10 Zellen/ml) von Kulturen von Candida albicans, welche 48 Stunden alt sind. Die Testsubstanzen (TS, Verbindungen der Formel I) werden in Konzentrationen von 80-1,25 $\mu$g/ml und die Sterol-Biosynthese-Hemmer (SBH) in Konzentrationen von 20-0,001 $\mu$g/ml appliziert, wobei die Verdünnungsschritte jeweils 1:2 betragen. Die Kulturen werden jeweils während 2 Tagen bei 37°C inkubiert. Man ermittelt dann die minimalen Hemmkonzentrationen (MIC) der verschiedenen Wirkstoffe bei der alleinigen und bei der kombinierten Applikation und berechnet aus den ermittelten MIC-Werten die fraktionierte Hemmkonzentration (FIC) nach folgender Formel:

$$FIC = \frac{\text{MIC (TS allein)}}{\text{MIC (TS in Kombination)}} + \frac{\text{MIC (SBH allein)}}{\text{MIC (SBH in Kombination)}}$$

Eine synergistische Wirkung liegt vor, wenn die FIC <0,5 ist. Die in der nachfolgenden Tabelle II enthaltenen Daten für die Verbindungen 14 und 20 gemäss Tabelle I, repräsentativen Vertretern der durch die Formel I definierten Verbindungsklasse, in Kombination mit Ketoconazol bzw. Terbinatin, repräsentativen Sterol-Biosynthese-Hemmern, belegen die synergistische Wirkung.

Tabelle II

| C.albicans | MIC in µg/ml Verbindung 20/Ketoconazol allein | | Verbindung 20/Ketoconazol in Kombination | | FIC |
|---|---|---|---|---|---|
| $H_{12}$ | 2 | 5 | 0,25 | 0,15 | 0,156 |
| | | | 0,12 | 0,075 | 0,078 |
| $H_{29}$ | 1 | 0,15 | 0,25 | 0,019 | 0,375 |
| 3153 | 1 | 2,5 | 0,12 | 0,075 | 0,156 |
| $B_5$ | 1 | 0,15 | 0,25 | 0,019 | 0,25 |
| $B_4$ | 2 | 2,5 | 0,12 | 0,075 | 0,093 |

| | MIC in µg/ml Verbindung 20/Terbinafin allein | | Verbindung 20/Terbinafin in Kombination | | FIC |
|---|---|---|---|---|---|
| $H_{12}$ | 2 | 6 | 0,25 | 0,75 | 0,25 |
| $H_{29}$ | 1 | 3,1 | 0,25 | 0,75 | 0,50 |
| 3153 | 1 | 6 | 0,12 | 1,50 | 0,375 |
| $B_5$ | 1 | 3,1 | 0,25 | 0,7 | 0,5 |
| $B_4$ | 2 | 100 | 0,50 | 1,5 | 0,266 |

Tabelle II - Fortsetzung

| C.albicans | MIC in µg/ml Verbindung 14/Ketoconazol allein | | MIC in µg/ml Verbindung 14/Ketoconazol in Kombination | | FIC |
|---|---|---|---|---|---|
| $H_{12}$ | 1 | 10 | 0,12 | 0,075 | 0,133 |
| | | | 0,06 | 0,6 | 0,125 |
| $H_{29}$ | 1 | 0,15 | 0,06 | 0,019 | 0,187 |
| | | | 0,12 | 0,038 | 0,375 |
| 3153 | 1 | 2,5 | 0,06 | 0,6 | 0,187 |
| | | | 0,12 | 0,075 | 0,156 |
| $B_5$ | 1 | 0,3 | 0,06 | 0,019 | 0,125 |
| | | | 0,12 | 0,038 | 0,25 |
| $B_4$ | 2 | 5 | 0,12 | 0,075 | 0,078 |
| | | | 0,06 | 0,15 | 0,040 |

| | MIC in µg/ml Verbindung 14/Terbinafin allein | | MIC in µg/ml Verbindung 14/Terbinafin in Kombination | | FIC |
|---|---|---|---|---|---|
| $H_{12}$ | 1 | 100 | 0,5 | 1,5 | 0,516 |
| $H_{29}$ | 1 | 25 | 0,25 | 3,0 | 0,375 |
| 3153 | 1 | 100 | 0,25 | 3,0 | 0,281 |
| | | | 0,25 | 1,5 | 0,561 |
| $B_5$ | 1 | 25 | 0,25 | 3,0 | 0,375 |
| $B_4$ | 2 | 100 | 1 | 3,0 | 0,531 |

Die Verbindungen der Formel I und ihre pharmazeutisch annehmbaren Säureadditionssalze können als Heilmittel, z.B. in Form pharmazeutischer Präparate zur enteralen, parenteralen oder topischen Applikation, Verwendung finden. Sie können beispielsweise peroral, z.B. in Form von Tabletten, Lacktabletten, Dragees, Hart- und Weichgelatinekapseln, Lösungen, Emulsionen oder Suspensionen, rektal, z.B. in Form von Suppositorien, parenteral, z.B. in Form von Injektionslösungen oder Infusionslösungen, oder topisch, z.B. in

Form von Salben, Crèmen oder Oelen, verabreicht werden.

Die Herstellung der pharmazeutischen Präparate kann in jedem Fachmann geläufiger Weise dadurch erfolgen, dass man die beschriebenen Verbindungen der Formel I und ihre pharmazeutisch annehmbaren Säureadditionssalze, gegebenenfalls in Kombination mit anderen therapeutisch wertvollen Stoffen, beispielsweise den erwähnten Sterol-Biosynthese-Hemmern, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Für die Kombination mit Verbindungen der Formel I geeignete Sterol-Biosynthese-Hemmer sind beispielsweise die systemisch, antifungal wirksamen Azole vom Typ des Miconazols, z.B. Ketoconazol, Itraconazol und Fluconazol, und die systemisch, antifungal wirksamen Allylamine vom Typ des Naftifins, z.B. Naftifin und Terbinafin.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole. Für topische Präparate eignen sich als Trägermaterialien Glyceride, halbsynthetische und synthetische Glyceride, hydrierte Oele, flüssige Wachse, flüssige Paraffine, flüssige Fettalkohole, Sterole, Polyäthylenglycole und Cellulosederivate.

Als pharmazeutische Hilfsstoffe kommen die üblichen Stabilisierungs, Konservierungs, Netz- und Emulgiermittel, Mittel zur Verbesserung der Konsistenz, Mittel zur geschmacklichen Verbesserung, Salze zur Veränderung des osmotischen Druckes, Puffersubstanzen, Lösungsvermittler, Färbe- und Ueberzugsmittel und Antioxidantien in Frage.

Die Dosierung der Verbindungen der Formel I kann, abhängig von den zu bekämpfenden pathogenen Pilzen, dem Alter und dem individuellen Zustand des Patienten und von der Applikationsweise, innerhalb weiter Grenzen variieren und ist natürlich in jedem einzelnen Fall den individuellen Gegebenheiten anzupassen. Zur Verhütung und Bekämpfung von topischen und systemischen Infektionen durch pathogene Pilze kommt für den erwachsenen Patienten im Falle der Monotherapie eine tägliche Dosis von etwa 0,01 g bis etwa 4 g, insbesondere etwa 0,05 g bis etwa 2 g in Betracht. Je nach Dosierung ist es dabei zweckmässig, die Tagesdosis in mehreren Dosierungseinheiten zu verabreichen. Im Falle der Kombinationstherapie kommt eine tägliche Dosis von etwa 0,01 g bis etwa 2 g, insbesondere etwa 0,02 bis etwa 1 g einer Verbindung der Formel I und von etwa 0,02 g bis etwa 0,2 g eines Sterol-Biosynthese-Hemmers in Betracht.

Die pharmazeutischen Monopräparate enthalten zweckmässigerweise etwa 10-1000 mg, vorzugsweise 50-500 mg einer Verbindung der Formel I. Die Kombinationspräparate enthalten zweckmässigerweise etwa 10-500 mg, vorzugsweise 20-250 mg einer Verbindung der Formel I und etwa 50-100 mg eines Sterol-Biosynthese-Hemmers.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

## Beispiel 1

a) Eine Mischung aus 100 g 4-Brombenzophenon, 25 g Aethylenglykol und 3,35 g p-Toluolsulfonsäure in 625 ml Benzol wird während 6 Tagen unter Rückfluss zum Sieden erhitzt, wobei das gebildete Wasser laufend entfernt wird. Das erhaltene Gemisch wird eingedampft, und der Festkörper wird aus Hexan umkristallisiert. Man erhält 85,9 g (74%) des Aethylenglykolketals von 4-Brombenzophenon mit einem Smp. von 58-62°C.

b) Eine Mischung aus 1,28 g Palladium(II)acetat und 6,64 g Triphenylphosphin in 120 ml Tetrahydrofuran wird während 0,5 Stunden unter Rückfluss zum Sieden erhitzt. Man versetzt mit 35 g des Aethylenglykolketals von 4-Brombenzophenon. Anschliessend gibt man zur siedenden Lösung langsam das aus 4,17 g Magnesium und 29,4 g 4-Bromtoluol in 180 ml Tetrahydrofuran erhaltene Grignardreagenz dazu. Nach Beendigung der Zugabe wird das Gemisch noch während 3 Stunden unter Rückfluss zum Sieden erhitzt. Das Gemisch wird eingedampft, und der Rückstand wird in 320 ml Aethanol und 290 ml 2N wässriger

Salzsäure aufgenommen. Man erhitzt während 4 Stunden zum Sieden und dampft das Gemisch dann ein. Der Rückstand wird aus Aethanol umkristallisiert, wobei man 26 g (83%) 4-Benzoyl-4'-methylbiphenyl mit einem Smp. von 124-125 °C erhält.

c) Ein Gemisch aus 26,1 g 4-Benzoyl-4'-methylbiphenyl, 18.6 g N-Bromsuccinimid und 0,18 g Azaisobutyronitril in 600 ml Tetrachlorkohlenstoff wird während 4 Stunden zum Sieden erhitzt. Die erhaltene braune Lösung wird filtriert und eingedampft. Der Rückstand wird aus Cyclohexan umkristallisiert. Man erhält 29,9 g (89%) 4'-(Brommethyl)-4-biphenylylphenylketon als farblose Kristalle mit einem Smp. von 110-112 °C.

d) 516 mg 4'-(Brommethyl)-4-biphenylylphenylketon werden in einer 33-proz. Lösung von Dimethylamin in Aethanol gelöst. Die Lösung wird über Nacht bei Raumtemperatur gerührt und dann eingedampft. Der Rückstand wird mit wässriger Natriumcarbonatlösung behandelt, und das Produkt wird mit Aether extrahiert. Die Extrakte werden über Magnesiumcarbonat getrocknet und mit einer 22-proz. Lösung von Chlorwasserstoff in Aether behandelt. Das ausgefallene Hydrochlorid wird abfiltriert und getrocknet. Man erhält 280 mg (54%) 4'-[(Dimethylamino)methyl] -4-biphenylylphenylketon-hydrochlorid als gelblichen Festkörper mit einem Smp. von 226-230 °C.

Beispiel 2

a) 3,0 g 4'-(Brommethyl)-4-biphenylylphenylketon wird in 100 ml Aethanol gelöst und mit 0,72 g N-Allylmethylamin und 1.17 g Kaliumcarbonat behandelt. Das Gemisch wird während 5 Stunden unter Rückfluss zum Sieden erhitzt, eingedampft und mit Aether extrahiert. Die Extrakte werden getrocknet und mit einer 22-proz. Lösung von Chlorwasserstoff in Aether behandelt. Das ausgefallene Produkt wird abfiltriert und getrocknet. Man erhält 3,22 g (68%) 4'-[(Allylmethylamino)methyl] -4-biphenylylphenylketon-hydrochlorid als farblosen Festkörper mit einem Smp. von 138-140 °C.

In analoger Weise werden die nachfolgend beschriebenen Verbindungen hergestellt:

b) 4'-[(Diallylamino)methyl]-4-biphenylylphenylketon als gelbliches Oel nach Reinigung durch Chromatographie an Kieselgel unter Verwendung von Hexan/Essigester 7:3 als Elutionsmittel. Ausbeute 53%. Massenspektrum: Spitzen unter anderem bei m/e 367 (M+, 19%), 271 (100%), 165 (22%).

c) 4'-[(Aethylmethylamino)methyl] -4-biphenylylphenylketon-hydrochlorid als farblosen Festkörper mit einem Smp. von 258 °C. Ausbeute 66%.

d) 4'-[(Methylamino)methyl] -4-biphenylylphenylketon-hydrochlorid als farblosen Festkörper mit einem Smp. von 267 °C. Ausbeute 55%.

e) 4'-[[[2-Butenyl]methylamino]methyl] -4-biphenylylphenylketon-hydrochlorid als farblosen Festkörper mit einem Smp. von 120 °C. Ausbeute 22%.

f) 4'-[[(2-Methylallyl)methylamino]methyl] -4-biphenylylphenylketon-hydrochlorid als farblosen Festkörper mit einem Smp. von 146 °C. Ausbeute 89%.

g) 4'-[(Propylamino)methyl] -4-biphenylylphenylketon-hydrochlorid als farblosen Festkörper mit einem Smp. von 260 °C. Ausbeute 58%.

h) 4'-[(Methylpropylamino)methyl] -4-biphenylylphenylketon-hydrochlorid als farblosen Festkörper mit einem Smp. von 154 °C. Ausbeute 26%.

Beispiel 3

a) 1,06 g Palladium(II)acetat und 5,48 g Triphenylphosphin werden in 100 ml Tetrahydrofuran gelöst. Man versetzt mit 29 g des Aethylenglykolketals von 4-Brombenzophenon und erhitzt das Gemisch unter Rückfluss zum Sieden. Anschliessend wird eine Lösung des aus 26,3 g 4-Aethylbrombenzol und 3,46 g Magnesium in 100 ml Tetrahydrofuran erhaltenen Grignard-Reagenzes langsam zur obigen siedenden Lösung gegeben. Man erhitzt noch während 3 Stunden unter Rückfluss zum Sieden und arbeitet dann wie in Beispiel 1b) beschrieben auf. Nach Chromatographie an Kieselgel mit Toluol/Hexan 7:3 als Elutionsmittel erhält man 19,15 g (70%) 4'-Aethyl-4-benzoyl-biphenyl als farblosen Festkörper mit einem Smp. von 106-108 °C.

b) 19 g 4'-Aethyl-4-benzoyl-biphenyl, 12,4 g N-Bromsuccinimid und 0,12 g Aza-isobutyronitril in 400 ml Tetrachlorkohlenstoff werden während 1,5 Stunden unter Rückfluss zum Sieden erhitzt. Nach Abfiltrieren des ausgefallenen Materials wird die Lösung eingedampft, und der Rückstand wird aus Cyclohexan umkristallisiert. Man erhält 20,7 g (85%) 4'-(1-Bromäthyl)-4-benzoyl-biphenyl als farblosen Festkörper mit einem Smp. von 132-134 °C.

c) In Analogie zu Beispiel 1d) erhält man daraus rac-4'-[1-(Dimethylamino)äthyl] -4-biphenylylphenylketon-hydrochlorid als gelblichen Festkörper mit einem Smp. von 239-241 °C. Ausbeute 57%.

In analoger Weise werden die nachfolgend aufgeführten Verbindungen hergestellt:

d) rac-4'-[1-(Allylmethylamino)äthyl] -4-biphenylylphenylketon-hydrochlorid als farblosen Festkörper mit einem Smp. von 88-92 °C. Ausbeute 35%.

e) 4'-[(Dimethylamino)methyl]-3'-methyl -4-biphenylylphenylketon-hydrochlorid als farblosen Festkörper mit einem Smp. von 221-222 °C. Ausbeute 15%.

f) rac-4'-[1-(Pyrrolidino)äthyl] -4-biphenylylphenylketon-hydrochlorid als farblosen Festkörper mit einem Smp. von 184 °C. Ausbeute 14%.

g) 4'-[[(2-Methylallyl)methylamino]methyl]-3'-methyl -4-biphenylylphenylketon-hydrochlorid als farblosen Festkörper mit einem Smp. von 107-109 °C. Ausbeute 13%.

Beispiel 4

a) 35 ml Nitrobenzol werden in einem Eisbad abgekühlt und dann nacheinander mit 5,2 g Aluminiumchlorid und 5,0 g 4-Methylbiphenyl behandelt. Das Gemisch wird auf Raumtemperatur gebracht und dann langsam mit 7,7 g 2,4-Dichlor-benzoylchlorid versetzt. Das Gemisch wird über Nacht bei Raumtemperatur gerührt, auf Wasser gegossen und mit Methylenchlorid extrahiert. Die Extrakte werden mit 2N Salzsäure und Wasser gewaschen, über Magnesiumsulfat getrocknet und eingedampft. Der Rückstand wird an Kieselgel mit Toluol/Essigester 9:1 chromatographiert. Man erhält 6,7 g (63%) 2,4-Dichlorphenyl-4'-methyl -4-biphenylylketon als farblosen Festkörper mit einem Smp. von 184-185 °C.

b) Ein Gemisch aus 5,0 g 2,4-Dichlorphenyl-4'-methyl -4-biphenylylketon, 2,7 g N-Bromsuccinimid und 20 mg Azaisobutyronitril in 70 ml Tetrachlorkohlenstoff wird während 4 Stunden unter Rückfluss zum Sieden erhitzt. Das ausgefallene Material wird abfiltriert, und das Filtrat wird eingedampft. Der Rückstand wird aus Toluol/Cyclohexan umkristallisiert. Man erhält 5,5 g (89%) 2,4-Dichlorphenyl-4'-brommethyl -4-biphenylylketon als farblosen Festkörper mit einem Smp. von 92 °C.

c) 1,0 g 2,4-Dichlorphenyl-4'-brommethyl -4-biphenylylketon und 20 ml einer 33-proz. Lösung von Dimethylamin in Aethanol werden während 4 Stunden zum Sieden erhitzt, worauf das Gemisch eingedampft wird. Der Rückstand wird in Aether aufgenommen und mit einer ätherischen Lösung von Chlorwasserstoff behandelt. Das ausgefallene Hydrochlorid wird abfiltriert und getrocknet. Man erhält 2,4-Dichlorphenyl-4'-[(dimethylamino)methyl] -4-biphenylylketon-hydrochlorid mit einem Smp. von 195-196 °C. Ausbeute 51%.

d) 4'-[(Dimethylamino)methyl] -4-biphenylyl-p-fluorphenylketon-hydrochlorid mit einem Smp. von 230 °C. Ausbeute 41%.

e) 4'-[(Dimethylamino)methyl] -4-biphenylyl-1-naphthylketon-hydrochlorid mit einem Smp. von 226 °C. Ausbeute 50%.

f) 4'-[(Dimethylamino)methyl] -4-biphenylyl-2-naphthylketon-hydrochlorid mit einem Smp. von 263 °C. Ausbeute 54%.

g) 4'-[(Dimethylamino)methyl] -4-biphenylyl-p-nitrophenylketon-hydrochlorid mit einem Smp. von 228 °C. Ausbeute 64%.

h) p-[[(4'-[(Dimethylamino)methyl] -4-biphenylyl]carbonyl]benzonitril-hydrochlorid mit einem Smp. von 243 °C. Ausbeute 59%.

i) p-Bromphenyl-4'-[(dimethylamino)methyl] -4-biphenylylketon-hydrochlorid mit einem Smp. von 253 °C. Ausbeute 48%.

j) 4'-[(Dimethylamino)methyl] -4-biphenylyl-p-jodphenylketon-hydrochlorid mit einem Smp. >250 °C. Ausbeute 48%.

k) 2-Chlor-4-nitrophenyl-4'-[(dimethylamino)methyl] -4-biphenylylketon-hydrochlorid mit einem Smp. von 223 °C. Ausbeute 63%.

l) 2-Brom-4-chlorphenyl-4'-[(dimethylamino)methyl] -4-biphenylylketon-hydrochlorid mit einem Smp. von 227 °C. Ausbeute 61%.

m) 2,4-Dibromophenyl-4'-[(dimethylamino)methyl]-4-biphenylylketon-hydrochlorid mit einem Smp. von 239-244 °C. Ausbeute 76%.

n) 2-Methoxyphenyl-4'-[(dimethylamino)methyl]-4-biphenylylketon-hydrochlorid mit einem Smp. von 146 °C. Ausbeute 57%.

o) 2,4-Difluorophenyl-4'-[(dimethylamino)methyl]-4-biphenylylketon-hydrochlorid mit einem Smp. von 235 °C. Ausbeute 48%.

p) 2-Methylphenyl-4'-[(dimethylamino)methyl]-4-biphenylylketon-hydrochlorid mit einem Smp. von 213 °C. Ausbeute 44%.

Beispiel 5

a) 1,0 g 2,4-Dichlorphenyl-4'-brommethyl -4-biphenylylketon, 1,5 ml N-Allylmethylamin und 0,84 g Kaliumcarbonat in 25 ml Aethanol werden während 4 Stunden unter Rückfluss zum Sieden erhitzt. Das Gemisch wird eingedampft, und der Rückstand wird mit Aether extrahiert. Die Extrakte werden über Magnesiumsulfat getrocknet und mit einer ätherischen Lösung von Chlorwasserstoff behandelt. Das ausgefallene Hydrochlorid wird abfiltriert und getrocknet. Man erhält 2,4-Dichlorphenyl-4'-[-(allylmethylamino)methyl] -4-biphenylylketon-hydrochlorid als gelblichen Festkörper mit einem Smp. von 175-176 °C. Ausbeute 56%.

In analoger Weise werden die nachfolgend beschriebenen Verbindungen hergestellt:

b) 4'-[(Allylmethylamino)methyl] -4-biphenylyl-p-fluorphenylketon-hydrochlorid mit einem Smp. von 152-153 °C. Ausbeute 74%.

c) 4'-[(Allylmethylamino)methyl] -4-biphenylyl-1-naphthylketon-hydrochlorid mit einem Smp. von 189-190 °C. Ausbeute 44%.

d) 4'-[(Allylmethylamino)methyl] -4-biphenylyl-2-naphthylketon-hydrochlorid mit einem Smp. von 197 °C. Ausbeute 60%.

e) 4'[(Allylmethylamino)methyl] -4-biphenylyl-p-nitrophenylketon-hydrochlorid mit einem Smp. von 154 °C. Ausbeute 52%.

f) p-[[4'-[(Allylmethylamino)methyl] -4-biphenylyl]carbonyl]benzonitril-hydrochlorid mit einem Smp. von 91-93 °C. Ausbeute 24%.

g) 4'-[[(Allylmethylamino)methyl] -4-biphenylyl-p-bromphenylketon-hydrochlorid mit einem Smp. von 185 °C. Ausbeute 44%.

h) 4'-[(Allylmethylamino)methyl] -4-biphenylyl-p-jodphenylketon-hydrochlorid mit einem Smp. von 200 °C. Ausbeute 30%.

i) 4'-[(Allylmethylamino)methyl] -4-biphenylyl-2-chlor-4-nitrophenylketon-hydrochlorid mit einem Smp. von 123-126 °C. Ausbeute 55%.

j) 2-Brom-4-chlorphenyl-4'-[(allylmethylamino)methyl] -4-biphenylylketon-hydrochlorid mit einem Smp. von 194-195 °C. Ausbeute 64%.

k) 2,4-Dibromphenyl-4'-[(Allylmethylamino)methyl]-4-biphenylylketon-hydrochlorid mit einem Smp. von 198-203 °C. Ausbeute 59%.

l) 2,4-Difluorophenyl-4'-[(Allylmethylamino)methyl]-4-biphenylylketon-hydrochlorid mit einem Smp. von 155%. Ausbeute 55%.

m) 2-Methylphenyl-4'-[(Allylmethylamino)methyl]-4-biphenylylketon-hydrochlorid mit einem Smp. von 157-159 °C. Ausbeute 44%.

n) 3-Methylphenyl-4'-[(Allylmethylamino)methyl]-4-biphenylylketon-hydrochlorid mit einem Smp. von 66 °C. Ausbeute 27%.

Beispiel 6

a) Ein Gemisch aus 10 g 4-Isopropenyl-biphenyl und 6,7 g Natriumazid in 50 ml Chloroform wird auf -5 °C abgekühlt. Eine Lösung von 20,6 g Trifluoressigsäure in 50 ml Chloroform wird so dazugegeben, dass die Temperatur unterhalb 0 °C bleibt. Man lässt das Reaktionsgemisch dann über Nacht bei Raumtemperatur stehen. Das Gemisch wird dann mit Eiswasser und 25-proz. Ammoniumhydroxid behandelt und dreimal mit Methylenchlorid extrahiert. Die Extrakte werden über Magnesiumsulfat getrocknet und eingedampft. Der wachsartige Rückstand wird in Pentan aufgenommen, worauf man von wenig unlöslichem Material abfiltriert. Nach Eindampfen des Filtrates erhält man 10,9 g (89%) 4-(1-Azido-1-methyl)äthylbiphenyl als gelbliches Oel.

b) 10,9 g 4-(1-Azido-1-methyl)äthylbiphenyl wird in Isopropanol gelöst und dann auf 70 °C erwärmt. Die Lösung wird dann mit kleinen Portionen an feuchtem Raney-Nickel behandelt, wobei eine starke Entwicklung von Stickstoff beobachtet werden kann. Nach Beendigung der Zugabe wird das Reaktionsgemisch noch während 0,5 Stunden bei 70 °C gehalten und dann filtriert. Das Filtrat wird zwischen 2N Salzsäure und Essigester verteilt. Man filtriert erneut, um unlösliches Material zu entfernen, und verwirft dann die organische Phase. Die wässrige Phase wird durch Zugabe von Natriumhydroxid alkalisch gestellt und dann dreimal mit Essigester extrahiert. Die organische Phase wird über Magnesiumsulfat getrocknet und eingedampft. Man erhält 7,08 g (73%) 4-(1-Amino-1-methyl)äthylbiphenyl als gelblichen, kristallinen Feststoff.

c) 4,3 g 4-(1-Amino-1-methyl)äthylbiphenyl werden in 40 ml Aceton gelöst und dann mit 2,7 ml Methyljodid und 7,5 g Kaliumcarbonat behandelt. Das Gemisch wird während 6 Stunden unter Rückfluss

zum Sieden erhitzt und dann eingedampft. Der Rückstand wird an Kieselgel mit Methylenchlorid/Methanol 98:2 und dann an Aluminiumoxid mit Hexan/Essigester 98:2 chromatographiert. Man erhält 2,06 g (28%) 4-[1-(Dimethylamino)-1-methyläthyl]biphenyl.

d) 0,9 g 4-[1-(Dimethylamino)-1-methyläthyl]biphenyl wird in 9 ml Schwefelkohlenstoff gelöst und auf 0 °C abgekühlt. Man versetzt mit 1,1 g Aluminiumchlorid und dann mit 0,54 ml Benzoylchlorid. Nach Beendigung der Zugabe wird das Gemisch während 8 Stunden auf 40 °C erwärmt. Man versetzt dann mit einer weiteren Portion von 0,54 ml Benzoylchlorid und erwärmt noch während 10 Stunden auf 40 °C. Das Reaktionsgemisch wird dann auf Eiswasser gegossen, durch Zugabe von Natriumhydroxid alkalisch gestellt und mit Essigester extrahiert. Die organische Phase wird getrocknet und eingedampft, und der Rückstand wird an Aluminiumhydroxid mit Essigester/Hexan (1:4 und 1:1) chromatographiert, wobei man 0,45 g eines unreinen Materials erhält. Dieses wird in Aether aufgenommen und mit einer ätherischen Lösung von Chlorwasserstoff behandelt. Das ausgefallene Hydrochlorid wird abfiltriert und getrocknet. Man erhält 0,42 g (29%) 4'-[1-(Dimethylamino)-1-methyläthyl] -4-biphenylylphenylketon-hydrochlorid als gelblichen Festkörper mit einem Smp. von 241 °C.

e) In analoger Weise erhält man p-Bromphenyl-4'-[1-(Dimethylamino)-1-methyläthyl] -4-biphenylylketon-hydrochlorid mit einem Smp. von 237 °C. Ausbeute 21%.

Beispiel 7

a) Eine Lösung des aus 344 mg Magnesium und 2,27 g 2-Bromtoluol in 15 ml Tetrahydrofuran hergestellten Grignard-Reagenzes wird tropfenweise zu einer Suspension von 2 g (±)-1-Dimethylamino-1-(4-bromphenyl)-äthan und 158 mg Tetrakistriphenylphosphinpalladium in 10 ml Tetrahydrofuran gegeben. Die Zugabe erfolgt dabei bei Raumtemperatur und unter einer Argonatmosphäre. Nach Beendigung der Zugabe wird das Gemisch noch während 5 Stunden zum Sieden erhitzt und dann unter vermindertem Druck eingedampft. Man versetzt dann mit 50 ml Aether und 50 ml gesättigter Ammoniumchloridlösung und trennt die wässrige Phase ab. Diese wird noch zweimal mit 50 ml Aether extrahiert. Die vereinigten organischen Auszüge werden mit gesättigter Natriumchloridlösung gewaschen, über Magnesiumsulfat getrocknet und eingedampft. Der Rückstand wird durch Chromatographie an Kieselgel unter Eluieren mit Methylenchlorid/Methanol 9:1 gereinigt. Man erhält 2,04 g (96%) (±)-4'-[1-(Dimethylamino)-äthyl] -2-methylbiphenyl als gelbes Oel.

b) Ein Gemisch aus 4,76 g (±)-4'-[1-(Dimethylamino)äthyl] -2-methylbiphenyl, 2,94 g Hexamethylentetramin und 30 ml Trifluoressigsäure wird während 5 Tagen unter Rückfluss zum Sieden erhitzt. Das Reaktionsgemisch wird dann konzentriert und mit 100 ml Eiswasser versetzt, worauf man wahrend 15 Minuten rührt, mit Natriumcarbonat basisch stellt und mit Aether extrahiert. Nach Eindampfen der ätherischen Extrakte und Chromatographieren des Rückstandes an Kieselgel mit Methylenchlorid/Methanol 9:1 als Elutionsmittel erhält man 2,8 g (52%) (±)-4-[4'-(1-(Dimethylamino)äthyl)phenyl] -2-methylbenzaldehyd als gelbes Oel.

c) Eine Lösung des aus 0,94 g Brombenzol und 146 mg Magnesium in 5 ml Tetrahydrofuran hergestellten Grignard-Reagenzes wird tropfenweise zu einer Lösung von 1,07 g (±)-4-[4'-(1-(Dimethylamino)äthyl)phenyl] -3-methylbenzaldehyd in 10 ml Tetrahydrofuran gegeben. Die Zugabe erfolgt dabei bei Raumtemperatur und unter einer Argonatmosphäre. Das Gemisch wird dann während 6 Stunden bei Raumtemperatur gerührt und dann mit 50 ml gesättigter Ammoniumchloridlösung hydrolysiert. Man extrahiert dreimal mit je 50 ml Aether, trocknet die Extrakte über Magnesiumsulfat und dampft sie ein. Man erhält 1,3 g eines Materials, das man durch Chromatographieren an Kieselgel mit Methylenchlorid/Methanol 9:1 als Elutionsmittel reinigt. Man erhält 1,1 g (80%) (RS)-4'-[(RS)-1-(Dimethylamino)äthyl] -2-methyl-α-phenyl-4-biphenylmethanol als farbloses Oel.

d) Eine Lösung von 406 mg Dimethylsulfoxid in 2 ml Methylenchlorid wird innerhalb von 5 Minuten zu einer Lösung von 327 mg Oxalylchlorid in 10 ml Methylenchlorid bei -70 °C gegeben. Das Reaktionsgemisch wird während 2 Minuten gerührt, worauf innerhalb von 5 Minuten eine Lösung von 810 mg (RS)-4'-[(RS)-1-(Dimethylamino)äthyl] -2-methyl-α-phenyl-4-biphenylmethanol in 5 ml Methylenchlorid dazugegeben wird. Man rührt noch während weiteren 15 Minuten und versetzt das Reaktionsgemisch dann bei -70 °C mit 1,18 g Triäthylamin. Man lässt das Reaktionsgemisch dann auf Raumtemperatur erwärmen und versetzt mit 50 ml einer 5-proz. wässrigen Lösung von Natriumcarbonat. Die wässrige Phase wird zweimal mit je 50 ml Methylenchlorid extrahiert. Die organischen Phasen werden vereinigt, mit 50 ml gesättigter Natriumchloridlösung gewaschen und über Magnesiumsulfat getrocknet. Nach Filtrieren und Eindampfen unter vermindertem Druck erhält man 780 mg eines Materials, das man zu einer heissen Lösung von 263 mg Fumarsäure in 5 ml Aethanol gibt. Das ausgefallene Fumarat wird aus Aethanol umkristallisiert. Man erhält 810 mg (75%) rac-4'-[1-(Dimethylamino)äthyl] -2-methyl-4-biphenylylphenyl-

keton-fumarat als farblosen Festkörper mit einem Smp. von 166-168 ° C.

Beispiel 8

a) In Analogie zu Beispiel 7a) erhält man:
aus 4-Jod-N,N-dimethylbenzylamin und 3-Bromtoluol das N,N,3'-Trimethyl-4-biphenylmethanamin als eine gelbe Flüssigkeit (Sdp. 180-185 ° C/20 Pa).
aus 4-Jod-N-allyl-N-methylbenzylamin und 3-Bromtoluol das N-Allyl-N,3'-dimethyl-4-biphenylmethanamin als eine farblose Flüssigkeit (Sdp. 185-190 ° C/20 Pa).

In Analogie zu Beispiel 6d) erhält man:
b) aus N,N,3'-Trimethyl-4-biphenylmethanamin und 2-Methylbenzoylchlorid das 4'-[(Dimethylamino)-methyl]-3-methyl -4-biphenylyl-o-tolyl-keton als eine leicht gelbe Flüssigkeit. Ausbeute 17%. Massenspektrum: Spitzen: u.a. bei m/e: 343 (M+, 42%), 299 (45%), 58 (100%);
c) aus N,N,3'-Trimethyl-4-biphenylmethanamin und 4-Brombenzoylchlorid das p-Bromphenyl-4'-[-(dimethylamino)methyl] -3-methyl-4-biphenylyl-keton als gelblichen Festkörper mit einem Smp. von 108-110 ° C;
d) aus N-Allyl-N,3'-dimethyl-4-biphenylmethanamin und Benzoylchlorid das 4'-[(Allylmethylamino)-methyl]-3-methyl -4-biphenylylphenyl-keton als gelbliche Flüssigkeit. Ausbeute 25%. Massenspektrum: Spitzen: u.a. bei m/e: 355 (M+, 38%), 285 (100%), 84 (41%);
e) aus N-Allyl-N,3'-dimethyl-4-biphenylmethanamin und 4-Brombenzoylchlorid das 4'-[(Allylmethylamino)-methyl]-3-methyl -4-biphenylyl-p-bromphenyl-keton und nach Behandeln mit Salzsäure in Aether das entsprechende Hydrochlorid als gelblichen Festkörper mit einem Smp. von 158-160 ° C. (Ausbeute 46%).

Beispiel 9

a) Zu einer Lösung von 5,43 g 4'-Methyl-biphenylcarbonsäurechlorid (Collect. Czech. Chem. Commun. 1978, 43, 257) und 2,53 g N,O-Dimethyl-hydroxylaminhydrochlorid in 50 ml Methylenchlorid unter Argon bei 0 ° C tropft man 6,15 g Pyridin innerhalb von 15 Minuten. Das Reaktionsgemisch wird bei Raumtemperatur während 1 Stunde gerührt, dann zweimal mit 50 ml einer gesättigten Natrium-bicarbonatlösung gewaschen. Die organische Phase wird über Magnesiumsulfat getrocknet und eingeengt. Der Rückstand wird bei Raumtemperatur und etwa 12 Pascal getrocknet. Man erhält 6 g (98%) N-Methoxy-N-methyl-4'-methyl-4-biphenylcarboxamid als farbloses Oel.
b) 4 g N-Methoxy-N-methyl-4'-methyl-4-biphenylcarboxamid, 3,34 g N-Bromsuccinimid und 0,02 g Azaisobutyronitril in 50 ml Tetrachlorkohlenstoff werden während 18 Stunden unter Rückfluss zum Sieden erhitzt. Man verdünnt das Reaktionsgemisch mit 50 ml Methylenchlorid und wäscht es zweimal mit je 50 ml Wasser und einmal lmit 50 ml gesättigter Natriumchloridlösung. Die organischen Phasen werden über Magnesiumsulfat getrocknet und eingeengt. Der Rückstand wird aus Essigester umkristallisiert. Man erhält 4,1 g (78%) 4'-Brommethyl-N-methoxy-N-methyl-4-biphenylcarboxamid als farblosen Festkörper mit einem Smp. von 105-106 ° C.
c) Man versetzt 1,95 g 4'-Brommethyl-N-methoxy-N-methyl-4-biphenylcarboxamid mit 20 ml einer 33-prozent. Lösung von Dimethylamin in Aethanol, rührt während 24 Stunden bei Raumtemperatur, dampft das Reaktionsgemisch ein, löst den Rückstand in 50 ml Essigester, wäscht mit 50 ml Natronlauge und mit 50 ml einer gesättigten Natriumchloridlösung. Die organischen Phasen werden über Magnesiumsulfat getrocknet und eingedampft. Das rohe Produkt wird an 100 ml Kieselgel mit Methylenchlorid/Methanol 9:1 chromatographiert. Man erhält 1,16 g (52%) 4'-[(Dimethylamino)methyl]-N-methoxy-N-methyl-4-biphenyl-carboxamid als farblosen Festkörper mit einem Smp. von 42-43 ° C.
d) Eine Lösung des aus 228 mg Magnesium und 1,42 g n-Pentylbromid in 10 ml Tetrahydrofuran hergestellten Grignardreagenzes wird tropfenweise zu einer Lösung von 1.16 g 4'-[Dimethylamino)-methyl]-N-methoxy-N-methyl-4-biphenylcarboxamid in 10 ml Tetrahydrofuran bei 0 ° C unter Argon zugetropft. Nach Beendigung der Zugabe wird das Gemisch noch während 5 Stunden bei Raumtemperatur gerührt und dann unter vermindertem Druck eingedampft. Man versetzt mit 50 ml Methylenchlorid und 50 ml gesättigter Ammoniumchloridlösung und trennt die wässrige Phase ab. Diese wird noch zweimal mit 50 ml Methylenchlorid extrahiert. Die vereinigten organischen Auszüge werden mit gesättigter Natriumchloridlösung gewaschen, über Magnesiumsulfat getrocknet und eingdampft. Der Rückstand wird durch Chromatographie an Kieselgel unter Eluieren mit Methylenchlorid/Methanol 95:5 gereinigt. Man erhält 0,95 g (80%) eines farblosen Oel. Dieses wird in Aether aufgenommen und mit einer ätherischen Lösung von Chlorwasserstoff behandelt. Das ausgefallene Hydrochlorid wird abfiltriert und getrocknet. Man erhält 0,83 g (61%) 4'-[(Dimethylamino)methyl]-4-biphenylyl-pentylketon-hydrochlorid

als farblosen Festkörper mit einem Smp. von 243-245 °C.

Beispiel 10

a) In Analogie zu Beispiel 9d) erhält man aus 4'-[(Dimethylamino)methyl]-N-methoxy-N-methyl-4-biphenylcarboxamid und 5-Bromomagnesium-2-methyl-2-penten das 4'-[(Dimethylamino)methyl]-4-biphenylyl-4-methyl-3-pentenyl-keton-hydrochlorid als farblosen Festkörper (Ausbeute 48%) mit einem Smp. von 230-232 °C.

b) In Analogie zu Beispiel 9d) erhält man aus 4'-[(Dimethylamino)methyl]-N-methoxy-N-methyl-4-biphenylcarboxamid und 2-[(8-Bromomagnesiumoctyl)oxy]tetrahydro-2H-pyran (J. Am. Chem. Soc. 1978, 100, 4878) das 4'-[(Dimethylamino)methyl]-biphenylyl-8-hydroxyoctylketonhydrochlorid als farblosen Festkörper mit einem Smp. von 190-191 °C. Ausbeute 34%.

Beispiel 11

a) In Analogie zu Beispiel 9c) erhält man aus 4'-Brommethyl-N-methoxy-N-methyl-4-biphenylcarboxamid und N-Allyl-N-methylamin das 4'-[(Allylmethylamino)methyl]-N-methoxy-N-methyl-4-biphenylcarboxamid als farblose Flüssigkeit (Ausbeute 65%).

$^1$H-NMR ($CDCl_3$): 2,25 (s, 3H), 3,06 (d, J = 6,5 Hz, 2H), 3,38 (s, 3H), 3,54 (s, 2H), 3,59 (s, 3H), 5,1-5,3 (m, 2H), 5,8-6,0 (m, 1H), 7,41 (d, J = 8 Hz, 2H), 7,5-7,8 (m, 6H).

b) In Analogie zu Beispiel 9d) erhält man aus 4'-[(Allylmethylamino)methyl]-N-methoxy-N-methyl-4-biphenylcarboxamid und n-Pentyl-magnesiumbromid das 4'-[(Allylmethylamino)methyl]-4-biphenylpentylketon-hydrochlorid als farblosen Festkörper (Ausbeute 51%) mit einem Smp. von 146-149 °C.

c) In Analogie zu Beispiel 10a) erhält man aus 4'-[(Allylmethylamino)methyl]-N-methoxy-N-methyl-4-biphenylcarboxamid und 5-Bromomagnesium-2-methyl-2-penten das 4'-[(Allyl-methylamino)methyl]-4-biphenylyl-4-methyl-3-pentenyl keton als leicht gelbliches Oel. Ausbeute 42%.

$^1$H-NMR ($CDCl_3$): 1,65, 1,70 (2s, 6H), 2,24 (s, 6H), 2,46 (q, J = 7 Hz, 2H), 3,05 (t, J = 7 Hz, 2H), 3,07 (d, J = 6,5 Hz, 2H), 3,57 (s, 2H), 5,1-5,3 (m, 3H), 5,8-6,0 (m, 1H), 7,42 (d, J = 8 Hz, 2H), 7,55 (d, J = 8 Hz, 2H), 7,67 (d, J = 8 Hz, 2H), 8,03 (d, J = 8Hz, 2H).

d) In Analogie zu Beispiel 10b) erhält man aus 4'-[(Allyl-methylamino)methyl]-N-methoxy-N-methyl-4-biphenylcarboxamid und 2-[8-Bromomagnesiumoctyl)oxy]tetrahydro-2H-pyran das 4'-[(Allylmethylamino)-methyl]-4-biphenylyl-8-hydroxyoctyl keton-fumarat als farblosen Festkörper mit einem Smp. von 84-85 °C. Ausbeute 46%.

Beispiel A

Die Verbindung 4'-[1-(Allylmethylamino)methyl]-4-biphenylyl -4-bromphenylketon kann wie folgt als Wirkstoff zur Herstellung von Tabletten verwendet werden:

| Bestandteile | mg/Tablette |
|---|---|
| Wirkstoff | 200 |
| Milchzucker pulv. | 100 |
| Povidone K 30 | 15 |
| Na-Carboxymethylstärke | 10 |
| Talk | 3 |
| Magnesiumstearat | 2 |
| Tablettengewicht | 330 |

Der Wirkstoff und der Milchzucker pulv. werden intensiv gemischt. Die erhaltene Mischung wird dann mit einer wässrigen Lösung von Povidone K 30 befeuchtet und geknetet, worauf man die erhaltene Masse granuliert, trocknet und siebt. Man mischt das Granulat mit den übrigen Bestandteilen und verpresst dann zu Tabletten geeigneter Grösse.

## Patentansprüche

1. Verbindungen der allgemeinen Formel

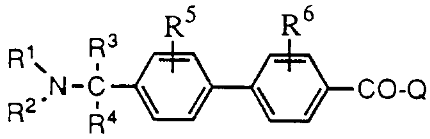

I

worin $R^1$ und $R^2$ je Wasserstoff, $C_{1-7}$-Alkyl oder $C_{2-7}$-Alkenyl oder zusammen geradkettiges $C_{2-4}$-Alkylen, $R^3$ und $R^4$ Wasserstoff oder $C_{1-7}$-Alkyl, $R^5$ und $R^6$ Wasserstoff, Halogen, Trifluormethyl, Nitro, Cyano, $C_{1-7}$-Alkoxy oder $C_{1-7}$-Alkyl; und Q eine unsubstituierte oder durch Halogen, Trifluormethyl, Cyano, Nitro, $C_{1-7}$-Alkyl oder $C_{1-7}$-Alkoxy einfach oder mehrfach substituierte Phenyl- oder Naphthylgruppe, oder eine $C_{1-10}$-Alkylgruppe, die durch eine oder gegebenenfalls mehrere Hydroxygruppen substituiert sein kann, oder eine $C_{1-10}$-Alkenylgruppe bedeuten,
und ihre pharmazeutisch annehmbaren Säureadditionssalze.

2. Verbindungen nach Anspruch 1, worin Q eine unsubstituierte oder durch Halogen, Trifluormethyl, Cyano, Nitro, $C_{1-7}$-Alkyl oder $C_{1-7}$-Alkoxy einfach oder mehrfach substituierte Phenyl- oder Naphthylgruppe bedeuten und worin $R^1$-$R^6$ die in Anspruch 1 angegebene Bedeutung haben.

3. Verbindungen nach Anspruch 1 oder 2, worin $R^1$ und $R^2$ $C_{1-4}$-Alkyl oder $C_{3-4}$-Alkenyl bedeuten.

4. Verbindungen nach Ansprüche 1 bis 3, worin $R^5$ und $R^6$ Wasserstoff, Halogen oder $C_{1-4}$-Alkyl bedeuten.

5. Verbindungen nach einem der Ansprüche 1 bis 4, worin Q eine unsubstituierte oder durch Halogen, Trifluormethyl, Nitro, Cyano oder $C_{1-4}$-Alkyl mono- oder disubstituierte Phenylgruppe oder eine $C_{5-10}$-Alkyl-, $C_{5-10}$-Hydroxyalkyl- oder $C_{5-10}$-Alkenylgruppe bedeutet.

6. 4'-[1-(Dimethylamino)äthyl]-4-biphenylylphenylketon,
   4-Bromphenyl-4'-[(dimethylamino)methyl]-4-biphenylylketon,
   4'-[(Dimethylamino)methyl-4-biphenylyl-4-jodphenylketon,
   4'-[1-(Allylmethylamino)äthyl]-4-biphenylylphenylketon,
   4'-[1-(Allylmethylamino)methyl]-4-biphenylyl-4-bromphenylketon,
   4'-[1-(Allylmethylamino)methyl]-4-biphenylyl-4-jodphenylketon.

7. 4'-[(Dimethylamino)methyl]-4-biphenylyl-4-methyl-3-pentenylketon,
   4'-[(Allylmethylamino)methyl]-4-biphenylyl-4-methyl-3-pentenylketon und
   4'-[(Allylmethylamino)methyl]-4-biphenylyl-8-hydroxyoctylketon.

8. Verbindungen der allgemeinen Formel

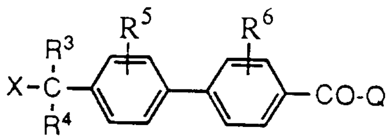

II

worin X eine Abgangsgruppe bedeutet, und $R^3$, $R^4$, $R^5$, $R^6$ und Q die in Anspruch 1 angegebene Bedeutung besitzen.

**9.** Verbindungen der allgemeinen Formel

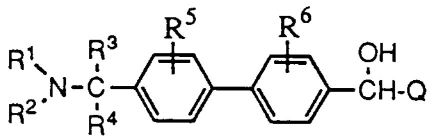

III

worin $R^1$, $R^2$, $R^3$, $R^4$, $R^5$, $R^6$ und Q die in Anspruch 1 angegebene Bedeutung besitzen.

**10.** Verbindungen der allgemeinen Formel

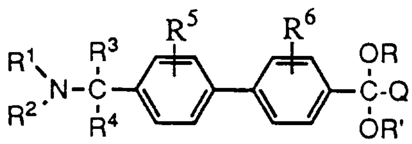

IV

worin $R^a$ und $R^b$ $C_{1-4}$-Alkyl oder zusammen Dimethylen oder Trimethylen bedeuten, und $R^1$, $R^2$, $R^3$, $R^4$, $R^5$, $R^6$ und Q die in Anspruch 1 angegebene Bedeutung besitzen.

**11.** Verbindungen der allgemeinen Formel

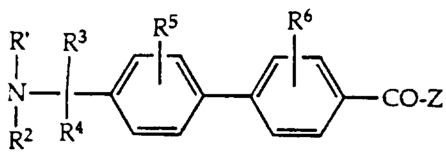

XXIV

worin $R^1$, $R^2$, $R^3$, $R^4$, $R^5$ und $R^6$ die in Anspruch 1 angegebene Bedeutung besitzen und Z eine Abgangsgruppe $NR^cR^d$ bedeutet, wobei $R^e$ nieder-Alkyl und $R^d$ nieder-Alkyl oder nieder-Alkoxy ist.

**12.** Verbindungen nach einem der Ansprüche 1 bis 7 zur Anwendung als therapeutische Wirkstoffe.

**13.** Verfahren zur Herstellung von Verbindungen nach einem der Ansprüche 1 bis 7 und von pharmazeutisch annehmbaren Säureadditionssalzen davon, dadurch gekennzeichnet, dass man

a) eine Verbindung der allgemeinen Formel

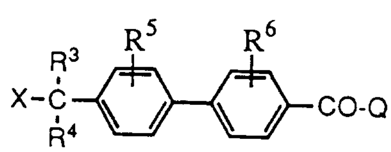

II

worin X eine Abgangsgruppe bedeutet, und $R^3$, $R^4$, $R^5$, $R^6$ und Q die in Anspruch 1 angegebene Bedeutung besitzen,

mit einem Amin der allgemeinen Formel $HNR^1R^2$, worin $R^1$ und $R^2$ die in Anspruch 1 angegebene Bedeutung besitzen, umsetzt, oder

b) eine Verbindung der allgemeinen Formel

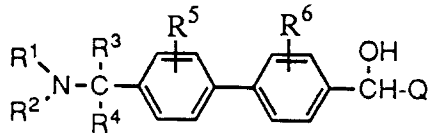

III

worin $R^1$, $R^2$, $R^3$, $R^4$, $R^5$, $R^6$ und Q die in Anspruch 1 angegebene Bedeutung besitzen, oxidiert, oder

c) eine Verbindung der allgemeinen Formel

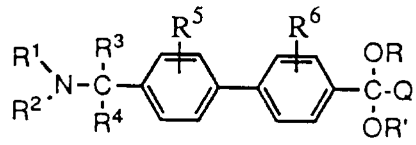

IV

worin $R^a$ und $R^b$ niederes Alkyl oder zusammen Dimethylen oder Trimethylen bedeuten, und $R^1$, $R^2$, $R^3$, $R^4$, $R^5$, $R^6$ und Q die in Anspruch 1 angegebene Bedeutung besitzen, mit einer wässrigen Säure behandelt, oder

d) eine Verbindung der allgemeinen Formel

X'-CO-Q V

worin X' Halogen bedeutet, und Q die in Anspruch 1 angegebene Bedeutung besitzt, in Gegenwart einer Lewis-Säure mit einer Verbindung der allgemeinen Formel

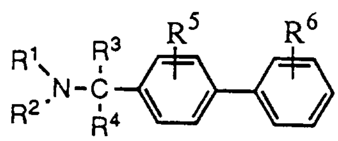

VI

worin $R^1$, $R^2$, $R^3$, $R^4$, $R^5$ und R' die in Anspruch 1 angegebene Bedeutung besitzen, umsetzt, oder

e) eine Verbindung der allgemeinen Formel

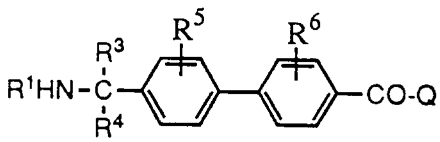

Ia

worin $R^1$, $R^3$, $R^4$, $R^5$, $R^6$ und Q die in Anspruch 1 angegebene Bedeutung besitzen, alkyliert oder alkenyliert,

f) eine Verbindung der allgemeinen Formel

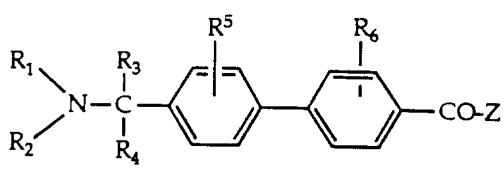

XXI

worin $R^1$, $R^2$, $R^3$, $R^4$, $R^5$, $R^6$ obige Bedeutung besitzen, und Z eine Abgangsgruppe der

allgemeinen Formel $NR^cR^d$ bedeutet, worin $R^c$ niederes Alkyl und $R^d$ niederes Alkyl oder Alkoxygruppe bedeuten, mit einer Verbindung der allgemeinen Formel M-Q worin M -MgCl, -MgBr, -MgI oder -Li bedeutet, und Q die obige Bedeutung besitzt,
umsetzt, und
g) eine erhaltene Verbindung der Formel I erwünschtenfalls in ein pharmazeutisch annehmbares Säureadditionssalz überführt.

**14.** Antimykotisch wirksame Mittel, enthaltend eine Verbindung gemäss einem der Ansprüche 1 bis 7 und einen therapeutisch inerten Träger, und gegebenenfalls eine bekannte antimykotisch wirksame Substanz, welche die Sterol-Biosynthese hemmt.

**15.** Verwendung von Verbindungen gemäss einem der Ansprüche 1 bis 7, gegebenenfalls in Kombination mit bekannten antimykotisch wirksamen Substanzen, welche die Sterol-Biosynthese hemmen, für die Herstellung von antimykotisch wirksamen Mitteln.

## Claims

**1.** Compounds of the general formula

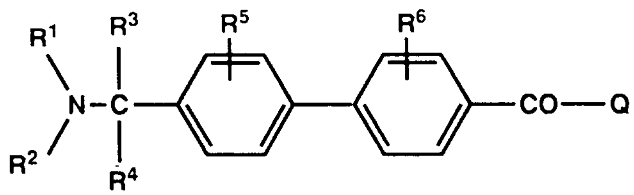

I

wherein $R^1$ and $R^2$ each signify hydrogen, $C_{1-7}$-alkyl or $C_{2-7}$-alkenyl or together signify straight-chain $C_{2-4}$-alkylene, $R^3$ and $R^4$ signify hydrogen or $C_{1-7}$-alkyl, $R^5$ and $R^6$ signify hydrogen, halogen, trifluoromethyl, nitro, cyano, $C_{1-7}$-alkoxy or $C_{1-7}$-alkyl and Q signifies a phenyl or naphthyl group, which is unsubstituted or mono- or multiply-substituted by halogen, trifluoromethyl, cyano, nitro, $C_{1-7}$-alkyl or $C_{1-7}$-alkoxy, or a $C_{1-10}$-alkyl group, which can be substituted by one or optionally several hydroxy groups, or a $C_{2-10}$-alkenyl group,
and their pharmaceutically acceptable acid addition salts.

**2.** Compounds according to claim 1, wherein Q signifies a phenyl or naphthyl group, which is unsubstituted or mono- or multiply-substituted by halogen, trifluoromethyl, cyano, nitro, $C_{1-7}$-alkyl or $C_{1-7}$-alkoxy, and wherein $R^1$-$R^6$ have the significance given in claim 1.

**3.** Compounds according to claim 1 or 2, wherein $R^1$ and $R^2$ signify $C_{1-4}$-alkyl or $C_{3-4}$-alkenyl.

**4.** Compounds according to claims 1 to 3, wherein $R^5$ and $R^6$ signify hydrogen, halogen or $C_{1-4}$-alkyl.

**5.** Compounds according to any one of claims 1 to 4, wherein Q signifies a phenyl group, which is unsubstituted or mono- or disubstituted by halogen, trifluoromethyl, nitro, cyano or $C_{1-4}$-alkyl, or a $C_{5-10}$-alkyl, $C_{5-10}$-hydroxyalkyl or $C_{5-10}$-alkenyl group.

**6.** 4'-[1-(Dimethylamino)ethyl]-4-biphenyly phenyl ketone,
4-bromophenyl 4'-[(dimethylamino)methyl]-4-biphenylyl ketone,
4'-[(dimethylamino)methyl]-4-biphenylyl 4-iodophenyl ketone,
4'-[1-(allylmethylamino)ethyl]-4-biphenylyl phenyl ketone,
4'-[1-(allylmethylamino)methyl]-4-biphenylyl 4-bromophenyl ketone,
4'-[1-(allylmethylamino)methyl]-4-biphenylyl 4-iodophenyl ketone.

**7.** 4'-[(Dimethylamino)methyl]-4-biphenylyl 4-methyl-3-pentenyl ketone,
4'-[(allylmethylamino)methyl]-4-biphenylyl 4-methyl-3-pentenyl ketone and
4'-[(allylmethylamino)methyl]-4-biphenylyl 8-hydroxyoctyl ketone.

**8.** Compounds of the general formula

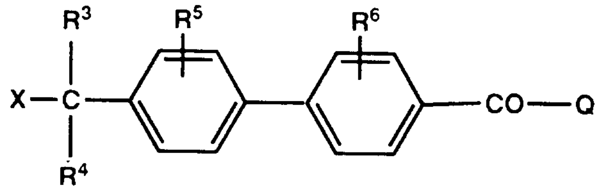

II

wherein X signifies a leaving group and $R^3$, $R^4$, $R^5$, $R^6$ and Q have the significance given in claim 1.

**9.** Compounds of the general formula

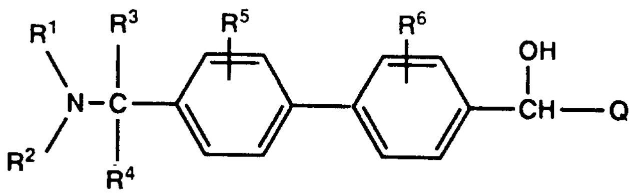

III

wherein $R^1$, $R^2$, $R^3$, $R^4$, $R^5$, $R^6$ and Q have the significance given in claim 1.

**10.** Compounds of the general formula

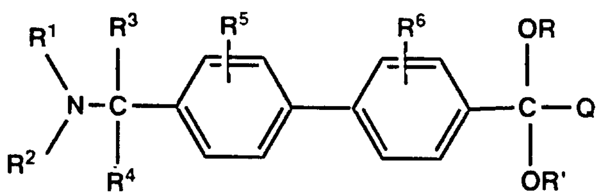

IV

wherein $R^a$ and $R^b$ signify $C_{1-4}$-alkyl or together signify dimethylene or trimethylene and $R^1$, $R^2$, $R^3$, $R^4$, $R^5$, $R^6$ and Q have the significance given in claim 1.

**11.** Compounds of the general formula

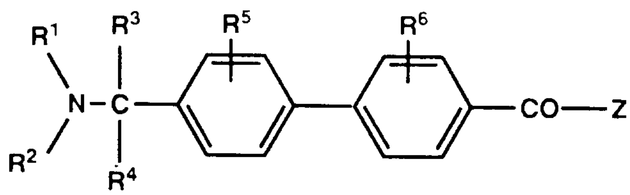

(XXIV)

wherein $R^1$, $R^2$, $R^3$, $R^4$, $R^5$ and $R^6$ have the significance given in claim 1 and Z signifies a leaving group $NR^cR^d$ in which $R^e$ signifies lower-alkyl and $R^d$ signifies lower-alkyl or lower-alkoxy.

**12.** Compounds according to any one of claims 1 to 7 for use as therapeutically active substances.

**13.** A process for the manufacture of compounds according to any one of claims 1 to 7 and of pharmaceutically acceptable acid addition salts thereof, characterized by

a) reacting a compound of the general formula

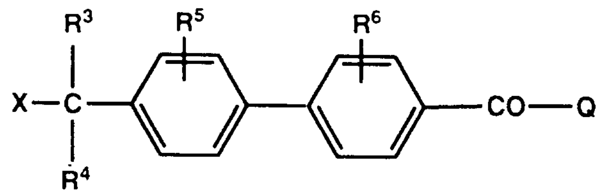 II

wherein X signifies a leaving group and $R^3$, $R^4$, $R^5$, $R^6$ and Q have the significance given in claim 1,
with an amine of the general formula $HNR^1R^2$, wherein $R^1$ and $R^2$ have the significance given in claim 1, or
b) oxidizing a compound of the general formula

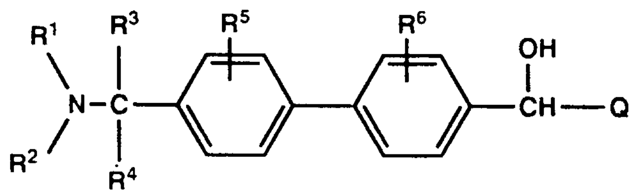 III

wherein $R^1$, $R^2$, $R^3$, $R^4$, $R^5$, $R^6$ and Q have the significance given in claim 1,
or
c) treating a compound of the general formula

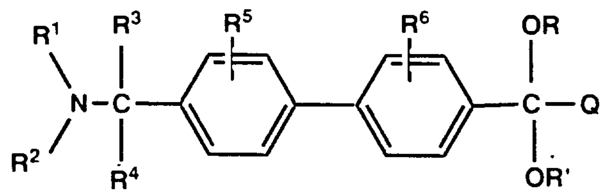 IV

wherein $R^a$ and $R^b$ signify lower alkyl or together signify dimethylene or trimethylene and $R^1$, $R^2$, $R^3$, $R^4$, $R^5$, $R^6$ and Q have the significance given in claim 1,
with an aqueous acid, or
d) reacting a compound of the general formula

X'—CO—Q V

wherein X' signifies halogen and Q has the significance given in claim 1,
in the presence of a Lewis acid with a compound of the general formula

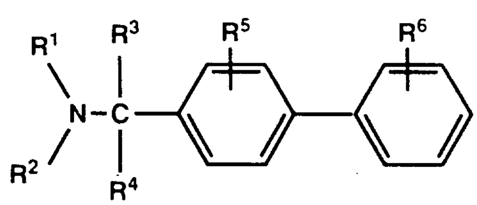 VI

wherein $R^1$, $R^2$, $R^3$, $R^4$, $R^5$ and $R^6$ have the significance given in claim 1,
or

e) alkylating or alkenylating a compound of the general formula

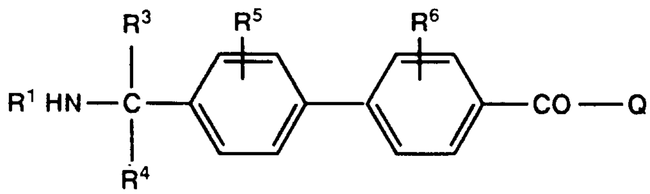

Ia

wherein $R^1$, $R^3$, $R^4$, $R^5$, $R^6$ and Q have the significance given in claim 1,

or

f) reacting a compound of the general formula

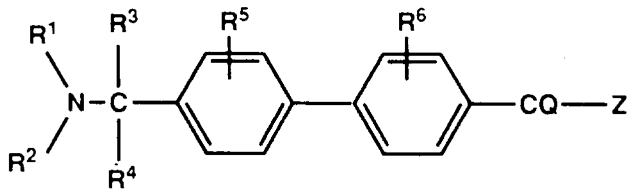

XXI

wherein $R^1$, $R^2$, $R^3$, $R^4$, $R^5$ and $R^6$ have the above significance and Z signifies a leaving group of the general formula $NR^cR^d$ in which $R^c$ signifies lower alkyl and $R^d$ signifies lower alkyl or lower alkoxy, with a compound of the general formula M-Q in which M signifies -MgCl, -MgBr, -MgI or -Li and Q has the above significance,

and

g) if desired, converting a compound of formula I obtained into a pharmaceutically acceptable acid addition salt.

**14.** An antimycotically-active medicament containing a compound in accordance with any one of claims 1 to 7 and a therapeutically inert carrier and optionally a known antimycotically-active substance which inhibits sterol biosynthesis.

**15.** The use of compounds in accordance with any one of claims 1 to 7, optionally in combination with known antimycotically-active substances which inhibit sterol biosynthesis, for the manufacture of antimycotically-active medicaments.

**Revendications**

**1.** Composés de formule générale

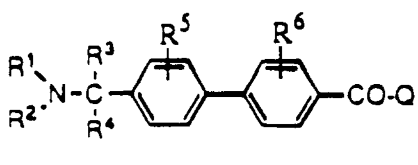

I

dans laquelle $R^1$ et $R^2$ représentent chacun i'hydrogène, un groupe alkyle en $C_1$-$C_7$ ou alcényle en $C_2$-$C_7$ ou bien forment ensemble un groupe alkylène à chaîne droite en $C_2$-$C_4$, $R^3$ et $R^4$ représentent l'hydrogène ou un groupe alkyle en $C_1$-$C_7$, $R^5$ et $R^6$ représentent l'hydrogène, un halogène, un groupe trifluorométhyle, nitro, cyano, alcoxy en $C_1$-$C_7$ ou alkyle en $C_1$-$C_7$ ; et Q représente un groupe phényle ou naphtyle non substitué ou portant un ou plusieurs substituants halogéno, trifluorométhlyle, cyano, nitro, alkyle en $C_1$-$C_7$ ou alcoxy en $C_1$-$C_7$, ou bien un groupe alkyle en $C_1$-$C_{10}$ qui peut être substitué par un ou éventuellement plusieurs groupes hydroxy, ou bien un groupe alcényle en $C_1$-$C_{10}$, et leurs sels formés par addition avec des acides acceptables pour l'usage pharmaceutique.

2. Composés selon revendication 1, pour lesquels Q représente un groupe phényle ou naphtyle non substitué ou portant un ou plusieurs substituants halogéno, trifluorométhyle, cyano, nitro, alkyle en $C_1$-$C_7$ ou alcoxy en $C_1$-$C_7$, et $R^1$ à $R^6$ ont des significations indiquées dans la revendication 1.

3. Composés selon revendication 1 ou 2, pour lesquels $R^1$ et $R^2$ représentent un groupe alkyle en $C_1$-$C_4$ ou alcényle en $C_1$-$C_4$.

4. Composés selon les revendications 1 à 3, pour lesquels $R^5$ et $R^6$ représentent l'hydrogène, un halogène ou un groupe alkyle en $C_1$-$C_4$.

5. Composés selon une des revendications 1 à 4, pour lesquels Q représente un groupe phényle non substitué ou mono- ou di-substitué par des halogènes, des groupes trifluorométhyle, nitro, cyano ou alkyle en $C_1$-$C_4$, ou bien un groupe alkyle en $C_5$-$C_{10}$, hydroxyalkyle en $C_5$-$C_{10}$ ou alcényle en $C_5$-$C_{10}$.

6. La 4'-[1-(diméthylamino)-éthyl]-4-biphénylylphénylcétone,
la 4-bromophényl-4'-[diméthylamino)-méthyl]-4-biphénylylcétone,
la 4'-[(diméthylamino)-méthyl-4-biphénylyl-4-iodophénylcétone
la 4'-[1-(allylméthylamino)-éthyl]-4-biphénylylphénylcétone,
la 4'-[1-(allylméthylamino)-méthyl]-4-biphénylyl-4-bromophénylcétone,
la 4'-[1-(allylméthylamino)-méthyl]-4-biphénylyl-4-iodophénylcétone.

7. La 4'-[(diméthylamino)-méthyl]-4-biphénylyl-4-méthyl-3-penténylcétone,
la 4'-[(allylméthylamino)-méthyl]-4-biphénylyl-4-méthyl-3-penténylcétone et
la 4'-[(allylméthylamino)-méthyl]-4-biphénylyl-8-hydroxyoctylcétone.

8. Composés de formule générale

II

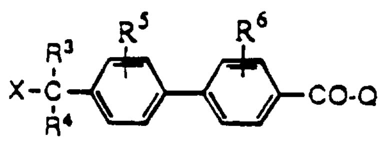

dans laquelle X représente un groupe éliminable et $R^3$, $R^4$, $R^5$, $R^6$ et Q ont les significations indiquées dans la revendication 1.

9. Composés de formule générale

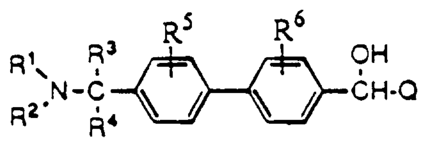

III

dans laquelle $R^1$, $R^2$, $R^3$, $R^4$, $R^5$, $R^6$ et Q ont les significations indiquées dans la revendication 1.

10. Composés de formule générale

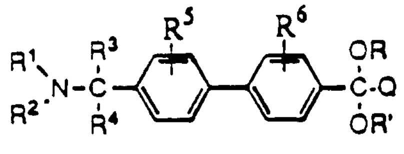

IV

dans laquelle $R^a$ et $R^b$ représentent des groupes alkyle en $C_1$-$C_4$ ou forment ensemble un groupe diméthylène ou triméthylène, et $R^1$, $R^2$, $R^3$, $R^4$, $R^5$, $R^6$ et Q ont les significations indiquées dans la revendication 1.

**11.** Composés de formule générale

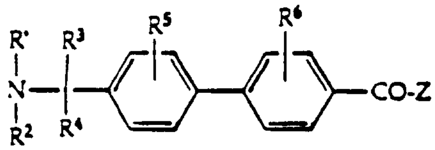

XXIV

dans laquelle $R^1$, $R^2$, $R^3$, $R^4$, $R^5$ et $R^6$ ont les significations indiquées dans la revendication 1 et Z représente un groupe éliminable $NR^cR^d$ dans lequel $R^c$ représente un groupe alkyle inférieur et $R^d$ un groupe alkyle inférieur ou alcoxy inférieur.

**12.** Composés selon l'une des revendications 1 à 7, pour l'utilisation en tant que substances thérapeutiques actives.

**13.** Procédé de préparation des composés selon une des revendications 1 à 7 et de leurs sels formés par addition avec des acides acceptables pour l'usage pharmaceutique, caractérisé en ce que

a) on fait réagir un composé de formule générale

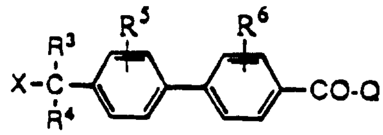

dans laquelle X représente un groupe éliminable et $R^3$, $R^4$, $R^5$, $R^6$ et Q ont les significations indiquées dans la revendication 1,

avec une amine de formule générale $HNR^1R^2$ dans laquelle $R^1$ et $R^2$ ont les significations indiquées dans la revendication 1, ou bien

b) on oxyde un composé de formule générale

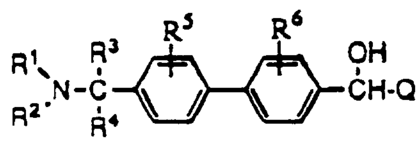

III

dans laquelle $R^1$, $R^2$, $R^3$, $R^4$, $R^5$, $R^6$ et Q ont les significations indiquées dans la revendication 1, ou bien

c) on traite par un acide aqueux un composé de formule générale

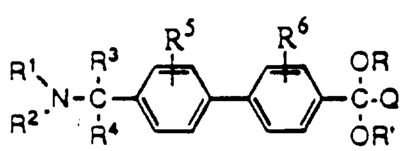

IV

dans laquelle $R^a$ et $R^b$ représentent des groupes alkyle inférieurs ou forment ensemble un groupe diméthylène ou triméthylène et $R^1$, $R^2$, $R^3$, $R^4$, $R^5$, $R^6$ et Q ont les significations indiquées dans la revendication 1, ou bien

d) on fait réagir un composé de formule générale

X'-CO-Q V

dans laquelle X' représente un halogène et Q a les significations indiquées dans la revendication 1, en présence d'un acide de Lewis, avec un composé de formule générale :

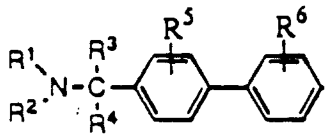

VI

dans laquelle $R^1$, $R^2$, $R^3$, $R^4$, $R^5$ et R' ont les significations indiquées dans la revendication 1, ou bien

e) on alkyle ou on alcényle un composé de formule générale

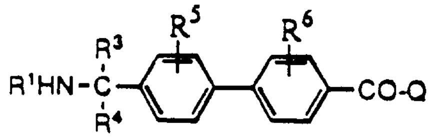

I a

dans laquelle $R^1$, $R^2$, $R^3$, $R^4$, $R^5$, $R^6$ et Q ont les significations indiquées dans la revendication 1,

f) on fait réagir un composé de formule générale

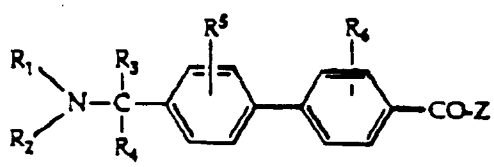

XXI

dans laquelle $R^1$, $R^2$, $R^3$, $R^4$, $R^5$, $R^6$ ont les significations indiquées ci-dessus et Z représente un groupe éliminable de formule générale $NR^cR^d$ dans laquelle $R^c$ représente un groupe alkyle inférieur et $R^d$ un groupe alkyle ou alcoxy inférieur, avec un composé de formule générale M-Q dans laquelle M représente -MgCl, - MgBr, -MgI ou -Li et Q a les significations indiquées ci-dessus, et

g) si on le désire, on convertit un composé ainsi obtenu, répondant à la formule I, en un sel par addition avec un acide acceptable pour l'usage pharmaceutique.

**14.** Produit antimycotique actif, contenant un composé selon l'une des revendications 1 à 7 et un véhicule inerte du point de vue thérapeutique, et le cas échéant une substance antimycotique active connue inhibant la biosynthèse des stérols.

**15.** Utilisation de composés selon l'une des revendications 1 à 7, éventuellement en combinaison avec des substancesantimycotiques actives connues inhibant la biosynthèse des stérols, pour la préparation de produits à activité antimycotique.